# EUROPÄISCHE PATENTSCHRIFT

(11) **EP 2 661 486 B1**
(45) Veröffentlichungstag und Bekanntmachung des Hinweises auf die Patenterteilung: **24.09.2014**
(21) Anmeldenummer: 12706764.3
(22) Anmeldetag: 06.01.2012
(51) Int. Cl.: C12M 3/00

(54) **PERFUSIONSBIOREAKTOR ZUM KULTIVIEREN VON ZELLEN AUF GERÜSTMATERIALIEN**
PERFUSION BIOREACTOR FOR CULTURING CELLS ON FRAMEWORK MATERIALS
BIORÉACTEUR À PERFUSION POUR LA CULTURE DE CELLULES SUR DES MATÉRIAUX DE STRUCTURE

(30) Priorität: 09.01.2011 DE 102011000061
(43) Veröffentlichungstag der Anmeldung: 13.11.2013
(73) Patentinhaber: Technische Universität Dresden, 01069 Dresden (DE)
(72) Erfinder: STIEHLER, Maik, 01445 Radebeul (DE); RAUH, Juliane, 01099 Dresden (DE); MILAN, Falk, 01309 Dresden (DE)
(74) Vertreter: Carlsohn, Alexander
(86) Internationale Anmeldenummer: PCT/DE2012/100004
(87) Internationale Veröffentlichungsnummer: WO 2012/092926

(56) Entgegenhaltungen:
- US-A- 3 682 318
- US-A- 5 576 211
- US-A1- 2004 110 273
- US-A1- 2004 253 716

## Beschreibung

Die Erfindung betrifft einen Perfusionsbioreaktor zum Kultivieren von Zellen auf Gerüstmaterialien sowie ein Perfusionsbioreaktorsystem, das einen solchen Perfusionsbioreaktor umfasst.

In dem Feld der Gewebszüchtung ("Tissue Engineering") werden Bioreaktoren unter anderem für die Herstellung von Ersatzgeweben eingesetzt (Ratcliffe et al., Bioreactors and bioprocessing for tissue engineering, Ann N Y Acad Sci., 2002, 5). Die Bioreaktoren sollen *in vitro* die Entwicklung von neuem Gewebe fördern, indem geeignete Stimulanzien, unter anderem Wachstumsfaktoren und dynamische Scherkräfte, während der Kultivierung der Gewebezellen bereitgestellt werden, wodurch - im Gegensatz zu zweidimensionalen Zellrasenkulturen - eine dynamische dreidimensionale Gewebekultur umgesetzt wird. Ein seit langem bekannter und einfach zu verwendender Bioreaktor ist die Spinnerflasche, die eine dreidimensionale Kulturvierung von Zellen auf porösen Mikroträgern ermöglicht. Dazu werden Gerüstmaterialien auf Nadeln aufgesteckt und in die mit einem Kulturmedium gefülltes Spinnerflasche eingebracht, an deren Boden ein Magnetrührer für eine Bewegung der Kulturlösung um die zellbesiedelten Gerüstmaterialien sorgt (siehe beispielsweise Stiehler et al., Effect of dynamic 3-D culture on proliferation, distribution, and osteogenic differentiation of human mesenchymal stem cells, J Biomed Mater Res A, 2008, 96, 98). Typischerweise wird die Kulturlösung mehrfach gewechselt, beispielsweise zweimal wöchentlich. Die Kultivierung in einer solchen Vorrichtung ist mit einer nicht-homogenen Durchströmung der zellulär besiedelten Gerüstmaterialien mit Zellkulturmedium sowie mit ebenso nicht-homogen wirkenden dynamischen Scherkräften innerhalb der Zell/Träger-Konstrukte verbunden.

Um eine einerseits gesicherte Durchdringung von Gerüstmaterialien mit Zellkulturmedium zu sichern und andererseits homogen über den gesamten Trägerstoff verteilt wirkende dynamische Scherkräfte zu erreichen, sind sogenannte Perfusionsbioreaktoren vorgeschlagen worden. In seiner einfachsten Form besteht ein solcher Bioreaktor aus einer rohrförmigen Kammer, in die die mit Zellen besiedelten, offenporigen Gerüstmaterialien eingebracht werden. Mittels einer Pumpe wird das Kulturmedium durch die Kammer gefördert, wodurch kontinuierlich Kulturmedium durch die Poren des Gerüstmaterials transportiert wird (siehe Bancroft et al., Design of a Flow Perfusion Bioreaktor System for Bone Tissue-Engineering Applications, Tissue Engineering, 2003, 9 (3), 549, 550). Wesentlich ist dabei, dass das Kulturmedium durch die untereinander vernetzten Poren der Gerüstmaterialen strömt und nicht etwa an Grenzflächen zwischen den Gerüstmaterialen und der Kammerwand und damit an den Gerüstmaterialien vorbei transportiert wird. Eine solche Führung der Strömung wird als "gezwungene Strömung" bezeichnet. Das Durchströmen der vernetzten Poren der Gerüstmaterialien ermöglicht eine vergleichsweise gute Versorgung der auf die Gerüstmaterialien aufgebrachten und in den Poren befindlichen Zellen, sowie einen Abtransport von Abbauprodukten, die von den Zellen produziert werden.

Zur Versorgung eines solchen Perfusionsbioreaktors mit Kulturmedium können ein erstes und ein zweites Medienreservoir vorgesehen sein. Mittels der Pumpe, beispielsweise einer Peristaltikpumpe, wird das Kulturmedium über eine Leitung von dem ersten Medienreservoir zu der Kulturkammer gepumpt, in die es durch eine Öffnung in deren oberer Stirnseite eintritt. In der Kulturkammer durchströmt das Kulturmedium wie beschrieben das dort befindliche zellulär besiedelte Gerüstmaterial und verlässt die Kulturkammer schließlich über eine Öffnung in deren unterer Stirnseite. Das Kulturmedium gelangt dann über eine weitere Leitung zu dem zweiten Medienreservoir, das über eine Leitung mit dem ersten Medienreservoir verbunden ist und den Austausch des umlaufenden Kulturmediums sowie einen Gasaustausch zwischen der Umgebungsluft und dem Kulturmedium ermöglichen soll (Bancroft et al., a. a. O., 552 f.).

Ein solches Perfusionsbioreaktorsystem ermöglicht ein dynamisches Kultivieren der Zellen auf den Gerüstmaterialien. Der Begriff "dynamisch" bezieht sich dabei auf die Tatsache, dass die Gerüstmaterialien durchströmt werden, während bei einer statischen Kultivierung keine Bewegung des Kulturmediums erzwungen wird. Das Perfusionsbioreaktorsystem gewährleistet eine ausreichende Versorgung der Zellen mit Sauerstoff und Nährstoffen und vermittelt zusätzlich wie beschrieben einen Stimulationsreiz zur zellulären Ausreifung.

Mittels Perfusionsbioreaktoren könnte eine Kultivierungsumgebung mit einer Strömung generiert werden, die den *in vivo* Bedingungen im menschlichen Körper ähnlich ist. Dies erfordert jedoch eine sorgfältige Einstellung der Kulturbedingungen sowie eine kontinuierliche Kontrolle dieser Bedingungen. Bekannte Perfusionsbioreaktoren sind zu diesem Zweck nicht hinreichend geeignet, da eine Kontrolle der Kulturbedingungen in der Kulturkammer nicht oder nur schwer möglich ist.

Volkmer et al. beschreiben in ihrem wissenschaftlichen Artikel "Hypoxia in Static and Dynamic 3D Culture Systems for Tissue Engineering of Bone" (Tissue Engineering, 2008, 14 (8), 1331, 1332 f.) einen Perfusionsbioreaktor mit gezwungener Perfusion, bei dem eine Sonde zur Messung des Sauerstoffpartialdrucks in die Kulturkammer eingeführt ist. Die Sondenspitze befand sich dabei im geometrischen Zentrum des in der Kulturkammer befindlichen Gerüstmaterials. Zum Einbringen der Sonde wurde ein Loch in die Kammerwand gebohrt. Für die alltägliche Praxis ist dieser Perfusionsbioreaktor daher nicht geeignet. Überdies ist eine Anpassung des Reaktors an Gerüstmaterialien mit verschiedenen Abmessungen nur mit hohem Aufwand möglich.

Perfusionsbioreaktoren sind zur Herstellung von Knochenersatzgeweben verwendet worden. Dabei werden Knochengerüstmaterialien (die auch als Trägermaterialien bezeichnet werden) mit Knochenvorläuferzellen besiedelt. Die Durchströmung der Gerüstmaterialien in dem Perfusionsbioreaktor ermöglicht eine knöcherne Ausreifung der Knochenvorläuferzellen. Für die klinische Anwendung zur Heilung größerer Knochendefekte ist allerdings der Einsatz von vitalen, großdimensionierten Knochengerüstmaterialen notwendig. Bekannte Perfusionsbioreaktoren sind jedoch aufgrund ihres Aufbaues nur zur Herstellung von vergleichsweise kleinen Knochenersatzgeweben geeignet.

In der klinischen Praxis ist es ferner erforderlich, Implantate, beispielsweise Knochenersatzgeweben, in verschiedensten Größen bereitzustellen. Es wäre überdies wünschenswert, wenn die Implantate Dimensionen aufwiesen, die unmittelbar von der Größe des zu heilenden Knochendefektes und nicht nur von der Größe der Kulturkammer des Bioreaktors bestimmt würde. Eine Anpassung bekannter Perfusionsbioreaktoren, um ein Implantat gewünschter Größe herzustellen, ist jedoch aufgrund des starren Aufbaues dieser Perfusionsbioreaktoren nicht oder nur schwer möglich. Beispielsweise sehen Bancroft et al, a. a. O., 552, die Verwendung von Rohren mit unterschiedlichen Durchmessern vor, die als Kassetten in die Kulturkammer eingesetzt werden sollen. Alternative Möglichkeiten, mittels Kombination simultan hergestellter kleinerer Knochenersatzgewebe große Gewebsdefekte zu behandeln, sind zudem mit dem Nachteil der damit verbundenen verminderten mechanischen Stabilität solcher kombinierten Konstrukte verbunden, die wiederum ein erhöhtes Risiko eines Implantatversagens darstellt.

Schließlich ist es eine elementare Forderung der klinischen Praxis, nur keimfreie Komponenten einzusetzen, was eine Sterilisation des Perfusionsbioreaktors sowie der weiteren Komponenten des Reaktorsystems erfordert. Hier ist eine möglichst einfache Sterilisierbarkeit des Perfusionsreaktorsystem wünschenswert, was bei bekannten Perfusionsreaktoren und -systemen nicht oder nur schwer möglich ist.

US 2004/0253716 A1 offenbart einen Bioreaktor zum Kultivieren von Zellen innerhalb einer Trägermatrix aus einem porösen Material. Der Bioreaktor weist ein inneres Gefäß auf, das in einem äußeren Gefäß angeordnet ist. Die Trägermatrix ist mit einer Zuführung für ein Kulturmedium ausgestattet, wozu im Träger der Matrix ein Einlass und ein Auslass ausgebildet sind.

US 2004/0110273 A1 offenbart eine Kulturkammer für biologische Präparate. In der Kulturkammer sind Membranen ausgebildet.

Weder der in US 2004/0253716 A1 beschriebene Bioreaktor noch die aus US 2004/0110273 A1 bekannte Kulturkammer sind zum Kultivieren größerer Gerüstmaterialen geeignet. Ferner erlauben sie keine Anpassung an unterschiedlich große Gerüstmaterialen.

Aufgabe der Erfindung ist es, die Nachteile nach dem Stand der Technik zu beseitigen. Es soll insbesondere ein Perfusionsreaktorsystem angegeben werden, das die Herstellung vergleichsweise großer Implantate, eine leichte Anpassung des Perfusionsreaktors an unterschiedlich große Gerüstmaterialien, eine kontinuierliche Überwachung der Kulturbedingungen in der Reaktorkammer, eine leichte Sterilisierbarkeit aller Komponenten des Systems sowie eine leichte Erweiterbarkeit, beispielsweise zur Integration weiterer technischer Funktionen, ermöglicht. Dabei sollen die Bedienbarkeit des Perfusionsreaktorsystems weder verschlechtert noch die Betriebs- und Herstellungskosten erhöht werden. Schließlich soll ein Verfahren zur Kultivierung von Zellen auf Gerüstmaterialien mittels des erfindungsgemäßen Perfusionsreaktorsystems angegeben werden.

Diese Aufgabe wird durch die Merkmale der Ansprüche 1 und 11 gelöst. Zweckmäßige Ausgestaltungen der Erfindungen ergeben sich aus den Merkmalen der Ansprüche 2 bis 10.

Nach Maßgabe der Erfindung ist ein Perfusionsbioreaktor zum Kultivieren von Zellen auf festen Gerüstmaterialien mit einer Kulturkammer zur Aufnahme des Gerüstmaterials vorgesehen, wobei
- die Kulturkammer in einem Gehäuse ausgebildet ist und die Mantelfläche der Kulturkammer von der Innenwand des Gehäuses gebildet ist und in den Stirnseiten des Gehäuses Gehäuseöffnungen für die Aufnahme von Verschlusselementen gebildet sind, die in die Gehäuseöffnungen dichtend einsteckbar sind,
- die Verschlusselemente kanalartige Öffnungen für die Zu- und Abfuhr eines Kulturmediums aufweisen und die kanalartige Öffnung eines oder beider Verschlusselemente einen Abschnitt aufweist, der sich, wenn das Verschlusselement in eine Gehäuseöffnung eingesteckt ist, von der Stirnseite des Verschlusselementes, die der Kulturkammer zugewandt ist, in Richtung der Stirnseite des Verschlusselementes, die der Kulturkammer abgewandt ist, verjüngt, und
- das Gehäuse einen ersten, zylinderförmigen Abschnitt und zweite, rohrförmige Abschnitte aufweist, die an die Stirnseiten des ersten, zylinderförmigen Abschnittes angrenzen, wobei in dem ersten, zylinderförmigen Anschnitt ein an dessen Stirnseiten offener Hohlraum ausgebildet ist, dessen Wandung die Mantelfläche der Kulturkammer bildet, und wobei die Verschlusselemente über die Gehäuseöffnungen in die zweiten, rohrförmigen Abschnitte einsteckbar sind.

Das Gehäuse ist vorzugsweise ein zylinderförmiges Gehäuse. Allerdings kann das Gehäuse auch jede andere äußere Form aufweisen. Nachstehend werden die Merkmale des erfindungsgemäßen Perfusionsbioreaktors am Beispiel eines Perfusionsbioreaktors mit zylinderförmigem Gehäuse beschrieben.

Der erfindungsgemäße Perfusionsbioreaktor ist modular aufgebaut, so dass eine Anpassung des Perfusionsbioreaktors an Gerüstmaterialien mit unterschiedlichen Abmessungen auf einfache Art und Weise möglich ist. Im Gegensatz zum Stand der Technik können Implantate hergestellt werden, deren Dimensionen deutlich über denen liegen, die mittels herkömmlicher Bioreaktoren hergestellt werden konnten. Ein Einbringen spezieller Kassetten in die Kulturkammer, mit denen ohnehin nur eine Anpassung an kleinere Gerüstmaterialien, nicht aber an größere Gerüstmaterialien möglich ist, ist bei dem erfindungsgemäßen Perfusionsbioreaktor nicht erforderlich.

Der erfindungsgemäße Bioreaktor ist zum Kultivieren von Zellen auf Gerüstmaterialien bestimmt. In der vorliegenden Erfindung wird unter einem Gerüstmaterial ein fester Körper verstanden. Dieser Körper ist dreidimensional. Er weist vorzugsweise eine offenporige Struktur auf, wobei die Poren in dem Körper vernetzt sein sollten. Das Gerüstmaterial kann mit Zellen besiedelt sein, bevor es in die Kulturkammer eingebracht wird. Der Ausdruck "Gerüstmaterial" umfasst in der vorliegenden Erfindung sowohl unbesiedelte als auch besiedelte Gerüstmaterialien.

Das Kulturmedium ist eine Nährlösung für die Zellen, die auf dem Gerüstmaterial in dem erfindungsgemäßen Perfusionsbioreaktor kultiviert werden sollen, und vorzugsweise eine Flüssigkeit oder ein Flüssigkeitsgemisch. Das Kulturmedium kann eine flüssige Lösung, eine Emulsion oder Dispersion sein. Das Kulturmedium sollte in der Kulturkammer in gezwungener Perfusion durch das Gerüstmaterial strömen, wobei Passieren der Kulturkammer ohne Durchströmen des Gerüstmaterials ausgeschlossen ist.

Die Kulturkammer ist in dem zylinderförmigen Gehäuse des erfindungsgemäßen Bioreaktorsystems ausgebildet, wobei die Innenwand des Gehäuses die Mantelfläche der Kulturkammer bildet. Unter einem zylinderförmigen Gehäuse wird hier ein Gehäuse mit zwei parallelen ebenen Flächen, den Stirnseiten, verstanden, die über eine Mantelfläche, die von parallelen Geraden gebildet ist, verbunden sind. In einer bevorzugten Ausführungsform hat das zylinderförmige Gehäuse im Wesentlichen die Form eines geraden Kreiszylinders. Das Gehäuse ist hohl, wobei einen Bereich des Hohlraumes die Kulturkammer bildet und an die Stirnseiten der Kulturkammer angrenzend weitere Bereiche des Hohlraums ausgebildet sind, in die die Verschlusselemente eingeführt sind und, falls vorhanden, Vermittlungselemente angeordnet sind.

Das Gehäuse des erfindungsgemäßen Perfusionsbioreaktors weist einen ersten, zylinderformigen Abschnitt und zweite, rohrförmige Abschnitte auf, die an die Stirnseiten des ersten, zylinderförmigen Abschnittes angrenzen, wobei in dem ersten, zylinderförmigen Abschnitt ein an dessen Stirnseiten offener Hohlraum ausgebildet ist, dessen Wandung die Mantelfläche der Kulturkammer bildet und die Verschlusselemente in die zweiten, rohrförmigen Abschnitte einsteckbar sind.

In einer bevorzugten Ausführungsform ist der erste, zylinderförmige Abschnitt ebenfalls rohrförmig. Allerdings ist dies nicht erforderlich. Da der erfindungsgemäße Bioreaktor vorteilhafterweise auch für die Herstellung von Implantaten geeignet ist, deren Mantelfläche nicht der eines einfachen geometrischen Körpers wie eines Quaders, Zylinders, Kegels oder Kegelstumpfes entspricht, sondern deren Mantelfläche in Abhängigkeit von den medizinischen Erfordernissen frei geformt sein kann, ist der Hohlraum nicht zwingend zylinder- oder kreiszylinderförmig. Vielmehr kann er eine beliebige Form aufweisen, solange er an seinen Stirnseiten, d. h. den Seiten, an denen die zweiten, rohrförmigen Abschnitte angrenzen, offen ist, so dass der Hohlraum in dem ersten Abschnitt in die Hohlräume in den zweiten Abschnitten übergeht. Ein solcher Hohlraum kann beispielsweise durch Auswahl einer geeigneten Gussform bei der Herstellung des Gehäuses erhalten werden. Alternativ kann das Gerüstmaterial in ein Silikonmaterial unter Erhalt eines zylinderförmigen, vorzugsweise kreiszylinderförmigen Körpers eingegossen werden, der dann wie ein zylinderförmiges Gerüstmaterial in das Gehäuse eingesetzt wird.

Sollen hingegen zylinderförmige Gerüstmaterialien in der Kulturkammer behandelt werden, ist der Hohlraum in dem ersten Abschnitt zweckmäßigerweise kreiszylinderförmig. Die sich an diesen kreiszylinderförmigen Hohlraum anschließenden kreiszylinderförmigen Hohlräume in den zweiten, rohrförmigen Abschnitten des Gehäuses weisen vorzugsweise einen größeren Durchmesser als der kreiszylinderförmige Hohlraum in dem ersten Abschnitt auf. Ist der Durchmesser zumindest einer der Gehäuseöffnungen gleich dem Durchmesser der kreiszylinderförmigen Hohlräume in den zweiten, ringförmigen Abschnitten, so kann das Gerüstmaterial über die Gehäuseöffnung und den angrenzenden zweiten Abschnitt in den ersten Abschnitt, dessen Hohlraum die Kulturkammer bildet eingefügt werden. In diesem Fall kann das Gehäuse einstückig ausgebildet sein.

Sollen mit dem erfindungsgemäßen Perfusionsbioreaktor hingegen Gerüstmaterialien behandelt werden, deren Durchmesser größer als der Durchmesser der Gehäuseöffnungen ist, so ist das Gehäuse zweckmäßigerweise zwei- oder mehrteilig. Im Falle eines zweitteiligen Gehäuses besteht das Gehäuse aus einem ersten und einem zweiten Gehäuseelement, die in einer Ebene, die die Kulturkammer schneidet, miteinander trennbar verbunden sind. Die Verbindung des ersten und des zweiten Gehäuseelementes kann beispielsweise mittels einer Schraub- oder Steckverbindung erfolgen. Zum Einbringen und Entnehmen eines Gerüstmaterials in die Kulturkammer kann das Gehäuse dann in seine beiden Teile zerlegt werden.

Die Größe der Kulturkammer ist zweckmäßigerweise so gewählt, dass die Mantelfläche eines Gerüstmaterials, das in die Kulturkammer eingebracht wird, unmittelbar an die Innenwand des Gehäuses, vorzugsweise die Wandung des ersten Abschnittes angrenzt, so dass eine erzwungene Perfusion des Kulturmediums ausschließlich durch das Gerüstmaterial erreicht wird.

Vorzugsweise liegen die Längsachsen des ersten, zylinderförmigen Abschnittes und der zweiten, rohrförmigen Abschnitte auf einer gemeinsamen Gerade, wobei die Außendurchmesser des ersten, zylinderförmigen Abschnittes und der zweiten, rohrförmigen Abschnitte gleich sind, so dass das Gehäuse eine Mantelfläche mit einheitlichem Außendurchmesser aufweist. Diese gemeinsame Gerade ist vorzugsweise die Längsachse des Gehäuses.

Die Ausdehnung der Kulturkammer in axialer Richtung, bezogen auf die Längsachse des Gehäuses, wird von der Längenausdehnung des zu behandelnden Gerüstmaterials bestimmt, wobei die Längenausdehnung des Gerüstmaterials geringer als die Summe der Ausdehnung der Hohlräume des ersten und der zweiten Abschnitte in axialer Richtung sein sollte. Ist dies der Fall, so kann der erfindungsgemäße Perfusionsbioreaktor leicht an die Behandlung von Gerüstmaterialien mit unterschiedlichen Längenausdehnungen angepasst werden, indem die Einstecktiefe der Verschlusselemente oder die Ausdehnung von Vermittlungselementen in axialer Richtung verändert wird. Die Ausdehnung der Kulturkammer und damit die Ausdehnung des ersten Abschnittes sowie der zweiten Abschnitte in axialer Richtung wird durch die Länge des Gerüstmaterials bestimmt und kann somit variieren, wobei die Summe der axialen Ausdehnung des ersten und der zweiten Abschnitte unveränderlich ist. Vorzugsweise entspricht die Ausdehnung der Kulturkammer und damit des ersten Abschnittes in axialer Richtung der Länge des Gerüstmaterials. Unter der Länge des Gerüstmaterials wird hier die Ausdehnung des Gerüstmaterials entlang einer Achse verstanden, die auf der Längsachse des Gehäuses und/oder des ersten Abschnittes liegt, wenn das Gerüstmaterial in die Kulturkammer eingebracht ist.

Die Ausdehnung der Kulturkammer in orthogonaler Richtung zu ihrer axialen Richtung wird durch die orthogonale Ausdehnung, bezogen auf die Längsachse des Gehäuses, des Hohlraums im ersten Abschnitt bestimmt. Eine Anpassung des erfindungsgemäßen Perfusionsbioreaktors in orthogonaler Richtung erfordert jedoch nur den Austausch des Gehäuses, nicht aber der Verschlusselemente, wenn die Durchmesser der Gehäuseöffnungen in den Stirnseiten des Gehäuses unverändert bleiben.

Erfindungsgemäß ist vorgesehen, dass in den Stirnseiten des Gehäuses Gehäuseöffnungen ausgebildet sind, in die Verschlusselemente dichtend eingesteckt werden können. Dazu weist zumindest eines der Verschlusselemente einen Einsteckkörper mit einer Außenfläche auf, die komplementär zu der Gehäuseöffnung ausgebildet ist und zumindest ein Dichtungselement zur Abdichtung der Grenzfläche zwischen der Außenfläche des Einsteckkörpers und der inneren Mantelfläche der Gehäuseöffnung trägt.

In einer bevorzugten Ausführungsform der Erfindung ist zumindest eines der Verschlusselemente zweiteilig ausgebildet, wobei das erste Teil ein im Wesentlichen zylinderförmiger Grundkörper ist, in dem die kanalartige Öffnung für die Zu- und Abfuhr eines Kulturmediums ausgebildet ist, und das zweite Teil ein Hülse ist, die auf dem Grundkörper lösbar befestigt ist.

In einer ersten Ausführungsform des zweiteiligen Verschlusselementes befindet sich die äußere Mantelfläche der Hülse in dichtendem Kontakt mit dem Gehäuse, wenn das Verschlusselement in das Gehäuse eingesteckt ist. Die äußere Mantelfläche der Hülse bildet in diesem Fall die Außenfläche des Einsteckkörpers. In einer zweiten Ausführungsform liegt ein Abschnitt der Mantelfläche des Grundkörpers frei, d. h. er wird nicht von der Hülse bedeckt, wenn diese auf dem Grundkörper befestigt ist. Zweckmäßigerweise grenzt dieser Abschnitt der Mantelfläche des Grundkörpers an die Stirnseite des Grundkörpers an, die der Kulturkammer zugewandt ist, wenn das Verschlusselement in die Gehäuseöffnung eingesteckt ist. In diesem Fall befindet sich der freiliegende Abschnitt des Grundkörpers in dichtendem Kontakt mit dem Gehäuse.

Die Hülse ist vorzugsweise ringförmig. Zur Befestigung der Hülse auf dem Grundkörper kann die Hülse in ihrer inneren Mantelfläche ein Innengewinde aufweisen, das komplementär zu einem Außengewinde ist, das an der äußeren Mantelfläche des Grundkörpers ausgebildet ist. Auf diese Weise können die Hülse und der Grundkörper miteinander verschraubt werden.

In der äußeren Mantelfläche der Hülse oder dem freiliegendem Abschnitt des Grundkörpers können in Umfangsrichtung zur Längsachse der Hülse umlaufende Nuten zur Aufnahme von Dichtungsringen vorgesehen sein. Die Nuten sind dabei in der äußeren Mantelfläche vorzugsweise in einem Bereich ausgebildet, der beim Einstecken der Verschlusselemente in die Gehäuseöffnungen in Kontakt mit der inneren Mantelfläche der Gehäuseöffnungen gelangt.

Weist der Grundkörper einen freiliegenden Abschnitt der Mantelfläche an seiner, der Kulturkammer zugewandten Stirnseite auf, wenn das Verschlusselement in das Gehäuse eingesteckt ist, so werden ein oder mehrere Dichtungselemente, beispielsweise Dichtungsringe, beim Verschrauben von Grundkörper und Hülse komprimiert, wodurch eine besonders dichter Verschluss der Gehäuseöffnung erreicht wird. Ohne dass dies zwingend erforderlich wäre, kann zu diesem Zweck vorgesehen sein, dass sich die Hülse beim Aufschrauben auf den Grundkörper in Richtung des freiliegenden Abschnittes bewegt, wodurch Druck auf die dort fixierten Dichtungselementen ausgeübt wird. Dabei werden die Dichtungselemente in axialer Richtung, bezogen auf die Längsachse des Grundkörpers komprimiert und dehnen sich radialer Richtung aus, wodurch ein dichter Verschluss erreicht werden kann.

Die Verschlusselemente können ferner Ausformungen, beispielsweise Riffelungen oder Kerbungen aufweisen, die ein Einstecken in die Gehäuseöffnungen und, im Falle eines Aufbaues des Verschlusselementes aus Hülse und Grundkörper, die ein Verschrauben von Hülse und Grundkörper erleichtern. Im letzteren Fall kann an der Hülse, an deren äußeren Mantelfläche ein umlaufender Steg ausgebildet sein, der regelmäßig Einkerbungen in seiner äußeren Mantelfläche aufweist. Ferner kann der Grundkörper Riffel aufweisen. Dies ist insbesondere dann zweckmäßig, wenn die Länge der Hülse geringer als die Länge des Grundkörpers, bezogen auf ihre Längsachse, ist, so dass ein Bereich der Mantelfläche des Grundkörpers, der an die Stirnseite des Grundkörpers angrenzt, freiliegt. In diesem Bereich können dann Riffelungen und/oder Kerbungen vorgesehen sein. Dieser Bereich ist dann ein zweiter freiliegenden Abschnitt der Mantelfläche des Grundkörpers, der an der anderen Stirnseite des Grundkörpers als der erste freiliegende Abschnitt ausgebildet ist.

Zumindest eines der Verschlusselemente, zweckmäßigerweise beide, weist eine kanalartige Öffnung für die Zu- und/oder Abfuhr des Kulturmediums mit einem Abschnitt auf, der sich, wenn das Verschlusselement in eine Gehäuseöffnung eingesteckt ist, von der Stirnseite des Verschlusselementes, die der Kulturkammer zugewandt ist, in Richtung der Stirnseite des Verschlusselementes, die der Kulturkammer abgewandt ist, verjüngt. Zweckmäßigerweise geht die kanalartige Öffnungen vor dem Erreichen der Stirnseite des Verschlusselementes, die der Kulturkammer abgewandt ist, in einem kreiszylinderförmigen Abschnitt über, der sich bis zu dieser Stirnseite erstreckt. Dieser Abschnitt weist vorzugsweise ein Innengewinde auf, in das ein rohrförmiges Anschlusselement für einen Schlauch derart eingeschraubt werden kann, dass ein Teil des Anschlusselementes aus dem Verschlusselement herausragt, wobei auf diesen Teil ein Schlauch aufgesteckt werden kann. Dazu kann dieser Teil in bekannter Weise einen Bereich mit einer sich verjüngenden äußeren Manteloberfläche aufweisen, der entlang der Längsachse des Anschlusselementes von einem umlaufenden Steg beabstandet ist, der so ausgeformt ist, dass er das Einschrauben des Anschlusselementes in das Verschlusselement erlaubt. Beispielsweise kann die äußere Mantelfläche des umlaufenden Stegs nach Art einer Schraube sechskantförmig ausgebildet sein.

Beide Verschlusselemente sind vorzugsweise gleich aufgebaut.

Eine oder beide Gehäuseöffnungen in den Stirnseiten des Gehäuses weisen vorzugsweise einen Durchmesser auf, der dem Innendurchmesser der zweiten Abschnitte entspricht. Allerdings können die Gehäuseöffnungen auch kleiner sein. Die Längsachsen der Gehäuseöffnungen liegen vorzugsweise auf der Längsachse des Gehäuses. Die Gehäuseöffnungen sind vorzugsweise gleich groß.

Zur Messung der Verfahrensbedingungen in der Kulturkammer und insbesondere in dem Gerüstmaterial, das sich in der Kulturkammer befindet, kann Sondenöffnung in dem Gehäuse vorgesehen sein. Die Sondenöffnung ist eine Öffnung in der Wandung des Gehäuses im Bereich der Kulturkammer, durch die eine Sonde in die Kulturkammer geführt werden kann. Ist der Hohlraum in dem ersten Abschnitt des Gehäuses zylinderförmig, so ist die Sondenöffnung vorzugsweise in der Mantelfläche des ersten Abschnittes derart ausgebildet, dass der Mittelpunkt der Sondenöffnung gleichmäßig von beiden Stirnseiten des ersten Abschnittes beabstandet ist. Die Sondenöffnung kann ein Innengewinde aufweisen, ist das die Sondenhalterung eingeschraubt werden kann.

In einer Ausführungsform der Erfindung umfasst der Perfusionsbioreaktor ferner ein Trägerelement, das auf das Gehäuse derart aufsteckbar ist, dass die Sonde durch eine Sondenöffnung in dem Gehäuse in die Kulturkammer eingeführt werden kann und dort sicher gehalten wird. Vorzugsweise weist das Trägerelement eine erste Bohrung, die komplementär zur Außenwand des Gehäuses zumindest im Bereich der Kulturkammer ist, und ein zweite Bohrung auf, deren Längsachse orthogonal zur Längsachse der ersten Bohrung verläuft, wobei eine Sondenhalterung durch die zweite Bohrung geführt ist. Die erste Bohrung sollte insbesondere komplementär zur äußeren Mantelfläche des ersten Abschnittes des Gehäuses sein. Die Sonde kann beispielsweise für die Messung des Sauerstoffpartialdruckes in dem Kulturmedium im Gerüstmaterial bestimmt sein.

Die Sondenhalterung ist vorzugsweise ein rohrförmiges Element, durch dessen Hohlraum eine stiftförmiger Messfühler der Sonde, beispielsweise eine Messelektrode, eine optische Faser oder anderes dem Fachmann bekanntes Messelement, verläuft. Die äußere Mantelfläche der Sondenhalterung weist vorzugsweise einen ersten Bereich auf, der komplementär zu der zweiten Bohrung in dem Trägerelement ausgebildet ist und sich, wenn die Sondenhalterung in das Trägerelement eingeführt ist, in Kontakt mit der Mantelfläche der zweiten Bohrung befindet. Auf diese Weise wird ein sicherer Halt der Sonde erreicht. Die äußere Mantelfläche der Sondenhalterung kann ferner einen zweiten Bereich aufweisen, der an den ersten Bereich angrenzt und so ausgeformt ist, dass er das Einstecken der Sondenhalterung in das Trägerelement erleichtert. Beispielsweise kann die Mantelfläche im zweiten Bereich nach Art einer Schraube sechskantförmig ausgebildet sein.

Zur Erleichterung der Positionierung des Trägerelementes kann in der Außenseite des Gehäuses eine Vertiefung vorgesehen sein, deren Kontur dem Außendurchmesser des ersten Bereiches der Sondenhalterung entspricht und in der die Sondenöffnung ausgebildet ist.

Ist das Trägerelement auf das Gehäuse aufgesteckt, so ist die Sondenhalterung derart durch die zweite Bohrung geführt, dass sich die äußere Mantelfläche des ersten Bereiches der Sondenhalterung in Kontakt mit der Mantelfläche der zweiten Bohrung befindet und eine Stirnseite der Sondenhalterung, zweckmäßigerweise die Stirnseite an die der erste Bereich angrenzt, in Kontakt mit der Außenseite des Gehäuse befindet. Ist eine Vertiefung in der Außenseite des Gehäuses vorgesehen, so grenzt die Stirnseite der Sondenhalterung an diese Vertiefung an. In einer Ausführungsform ist zusätzlich zwischen der Stirnseite der Sondenhalterung und der Außenseite des Gehäuses ein Dichtungsring angeordnet, der die Sondenöffnung gegen das Gehäuseäußere abdichtet.

Der Messfühler der Sonde ist vorzugsweise gegen die Gehäusewand sowie gegen die Außenbereiche des Gerüstmaterials, das sich in der Kulturkammer befindet, mittels eines Schutzelementes, beispielsweise einem Silikonschlauch, geschützt, der sich von Außenseite des Gehäuses bis zu einem Bereich erstreckt, der an die Messfühlerspitze angrenzt, die Messfühlerspitze aber nicht bedeckt. Das Schutzelement erleichtert ferner die Einführung des Messfühlers durch die Sondenöffnung in die hierfür vorgesehene Bohrung des in der Kulturkammer befindlichen Gerüstmaterials.

Die Messung des Sauerstoffpartialdruckes in dem Gerüstmaterial soll vorzugsweise in dessen geometrischer Mitte erfolgen. Ist das Gerüstmaterial in die Kulturkammer eingebracht, so kann der Messfühler der Sonde über die Sondenöffnung gemeinsam mit dem Schutzelement eingeführt werden, beispielsweise soweit, bis die Messfühlerspitze sich in der geometrische Mitte des Gerüstmaterials befindet. Nach der Behandlung des Gerüstmaterials in der Kulturkammer und vor der Entnahme des Gerüstmaterials aus der Kulturkammer wird die Sonde dann aus dem Trägerelement herausgezogen.

In einer Ausführungsform der Erfindung sind in dem Gehäuse ferner ein oder mehrere ringförmige Vermittlungselemente vorgesehen, die jeweils zwischen einer Stirnseite der Kulturkammer und dem dieser Stirnseite zugewandten Verschlusselemente angeordnet sind. Die Vermittlungselemente dienen zur Führung des Kulturmediums zwischen der Stirnseite der Kulturkammer und den kanalartigen Öffnungen der Verschlusselemente. Sie sind insbesondere dann zweckmäßig, wenn der Durchmesser der kanalartigen Öffnung an der Stirnseite eines Verschlusselementes, die der Kulturkammer zugewandt ist, geringer als der Durchmesser des Gerüstmaterials in der Kulturkammer ist und/oder wenn diese Stirnseite des Verschlusselementes von dem Gerüstmaterial, das sich in dem Kulturkammer befindet, derart beabstandet ist, dass tote Winkel in dem Gehäuse entstehen, die vom de Kulturmedium nicht laminar durchströmt werden. Auf diese Weise wird das Strömungsverhalten des Kulturmediums in der Kulturkammer verbessert. Zweckmäßigerweise sind die Vermittlungselemente in den zweiten Abschnitten des Gehäuses angeordnet.

Vorzugsweise ist vorgesehen, dass sich der Innendurchmesser eines Vermittlungselementes von der Stirnseite der Kulturkammer in Richtung des zugewandten Verschlusselementes verjüngt, wobei der Innendurchmesser des ringförmigen Vermittlungselementes an der Stirnseite, die an die Stirnseite der Kulturkammer angrenzt, dem dortigen Durchmesser der Kulturkammer und an der Stirnseite, die an das Verschlusselement angrenzt, dem dortigen Durchmesser der kanalartigen Öffnung entspricht.

Vorzugsweise bestehen sämtliche Bestandteile des erfindungsgemäßen Perfusionsbioreaktors aus einem autoklavierbaren Kunststoffmaterial. Alternativ sollten das Gehäuse, die Verschlusselemente und, falls vorgesehen, die Dichtungen und Vermittlungselemente aus einem autoklavierbaren Kunststoffmaterial gefertigt sein. Besonders bevorzugt sind auch das Trägerelement, die Sondenhalterung (einschließlich der Dichtung), das Schutzelement und die Anschlusselemente aus einem autoklavierbaren Kunststoffmaterial gefertigt werden. Ein geeignetes Kunststoffmaterial, aus denen die Dichtungen und/oder die Vermittlungselemente gefertigt sein können, ist ein Methyl-Vinyl-Silikon wie VMQ70 der Eriks GmbH, Bielefeld, DE. Ein geeignetes Kunststoffmaterial aus dem die übrigen Bestandteile gefertigt sind, ist ein Polyoxymethylen-Copolymer wie POM-C der KTK Kunststofftechnik Vertriebs GmbH, Germering, DE.

Nach Maßgabe der Erfindung ist ferner ein Perfusionsbioreaktorsystem vorgesehen, das neben dem erfindungsgemäßen Perfusionsbioreaktor, eine Pumpe zur Förderung eines Kulturmediums durch den Perfusionsbioreaktor sowie zumindest eine Durchfluss-Messzelle, beispielsweise zur Messung des Sauerstoffpartialdrucks in dem Kulturmedium, umfasst, wobei die Durchfluss-Messzelle unmittelbar vor oder nach dem Perfusionsbioreaktor, bezogen auf die Strömungsrichtung des Perfusionsbioreaktorsystems, angeordnet ist. Das erfindungsgemäße Perfusionsbioreaktorsystem kann ferner weitere Komponenten wie Vorratsbehälter, Abfallbehälter, Ausgleichs- und Probennahmebehälter sowie Ventile umfassen. Die Komponenten sind über Schläuche miteinander verbunden.

Der erfindungsgemäße Perfusionsbioreaktor und das erfindungsgemäße Perfusionsbioreaktorsystem sind insbesondere zur Herstellung von Implantaten, beispielsweise Knochenersatzgeweben, in verschiedensten Größen geeignet. Sie ermöglichen insbesondere Implantate herzustellen, die unmittelbar von der Größe des zu heilenden Knochendefektes und nicht nur von der Größe der Kulturkammer des Bioreaktors bestimmt werden. Soll ein Knochenersatzgewebe hergestellt werden, so wird ein Gerüstmaterial in die Kulturkammer eingebracht, das mit Knochenvorläuferzellen besiedelt ist. Nähere Angaben zu geeigneten Gerüstmaterialien, den Knochenvorläuferzellen sowie den Verfahrensbedingungen zur Kultivierung des mit den Knochenvorläuferzellen besiedelten Gerüstmaterials in der Kulturkammer lassen sich dem Stand der Technik entnehmen, beispielsweise Stiehler et al., Bancroft et al., und Volkmer et al., jeweils a.a.O. Ein für diesen Zweck besonders geeignetes Gerüstmaterial ist ein dreidimensionaler poröser Schaum, beispielsweise ein Schaum, Poly(D,L-milchsäure-coglycolsäure) (PLGA). Besonders geeignete Knochenvorläuferzellen sind mesenchymale Stammzellen (MSCs).

Die Kulturkammer wird im Stand der Technik gelegentlich auch als Reaktionskammer bezeichnet, ohne dass die Verwendung des Begriffes Kulturkammer in der vorliegenden Erfindung eine Unterscheidung zu einer Reaktionskammer treffen soll. Der Begriff "Kulturkammer" wurde gewählt, weil die Zellen auf dem Gerüstmaterial kultiviert werden.

Der erfindungsgemäße Perfusionsbioreaktor und das erfindungsgemäße Perfusionsbioreaktorsystem zeichnen sich insbesondere durch folgende Vorteile aus:
(a) Der erfindungsgemäße Perfusionsbioreaktor ermöglicht eine dynamische Kultivierung mit gezwungener Perfusion von großdimensionierten Gewebeersatzmaterialien.
(b) Eine Integration von invasiven und nicht invasiven Sensoren in den Perfusionsbioreaktor ist leicht möglich.
(c) Eine leichte Anpassbarkeit des Perfusionsbioreaktors, insbesondere der Kulturkammer, an unterschiedlich große Gerüstmaterialien und unterschiedliche Kultivierungsverfahren wird erreicht.
(d) Der Einsatz von Materialien gemäß der Good Medical Practice (GMP) und Fertigung aus Kunststoff und Silikon sowie der Verzicht auf Metallteile ermöglicht unter anderem eine problemlose Kombination mit anderen physikalischen Stimuli, z. B. elektromagnetischen Pulsen.
(e) Die Verwendung von Kunststoffen gewährleistet eine kostengünstige Fertigung bei größeren Stückzahlen.
(f) Die verwendeten Materialien ermöglichen die Sterilisation des Systems im Autoklaven.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine maßstabsgerechte Schnittdarstellung einer ersten Ausführungsform eines erfindungsgemäßen Perfusionsbioreaktors;
- Fig. 2: eine maßstabsgerechte perspektivische Explosionsdarstellung der in Fig. 1 gezeigten ersten Ausführungsform des erfindungsgemäßen Perfusionsbioreaktors;
- Fig. 3: eine maßstabsgerechte Seitenansicht der in Fig. 1 gezeigten ersten Ausführungsform des erfindungsgemäßen Perfusionsbioreaktors;
- Fig. 4: eine maßstabsgerechte Darstellung einer zweiten Ausführungsform eines erfindungsgemäßen Perfusionsbioreaktors (Fig. 4a: Draufsicht; Fig. 4b: Schnittdarstellung entlang Schnittachse A--A von Fig. 4a);
- Fig. 5: eine maßstabsgerechte Darstellung einer dritten Ausführungsform eines erfindungsgemäßen Perfusionsbioreaktors (Fig. 5a: Draufsicht; Fig. 5b: Schnittdarstellung entlang Schnittachse A--A von Fig. 5a);
- Fig. 6: eine maßstabsgerechte Darstellung einer vierten Ausführungsform eines erfindungsgemäßen Perfusionsbioreaktors (Fig. 6a: Draufsicht; Fig. 6b: Schnittdarstellung entlang Schnittachse A--A von Fig. 6a);
- Fig. 7: eine maßstabsgerechte Darstellung einer fünften Ausführungsform eines erfindungsgemäßen Perfusionsbioreaktors (Fig. 7a: Draufsicht; Fig. 7b: Schnittdarstellung entlang Schnittachse A--A von Fig. 7a);
- Fig. 8: eine maßstabsgerechte Darstellung einer sechsten Ausführungsform eines erfindungsgemäßen Perfusionsbioreaktors (Fig. 8a: Draufsicht; Fig. 8b: Schnittdarstellung entlang Schnittachse A--A von Fig. 8a);
- Fig. 9: eine maßstabsgerechte Darstellung einer siebenten Ausführungsform eines erfindungsgemäßen Perfusionsbioreaktors (Fig. 9a: Draufsicht; Fig. 9b: Schnittdarstellung entlang Schnittachse A--A von Fig. 9a);
- Fig. 10: eine maßstabsgerechte Darstellung einer achten Ausführungsform eines erfindungsgemäßen Perfusionsbioreaktors (Fig. 10a: Draufsicht; Fig. 10b: Schnittdarstellung entlang Schnittachse A--A von Fig. 10a);
- Fig. 11: eine maßstabsgerechte Darstellung einer neunten Ausführungsform eines erfindungsgemäße Perfusionsbioreaktors (Fig. 11a: Draufsicht; Fig. 11b: Schnittdarstellung entlang Schnittachse A--A von Fig. 11a);
- Fig. 12: eine maßstabsgerechte Darstellung einer zehnten Ausführungsform eines erfindungsgemäßen Perfusionsbioreaktors (Fig. 12a: Draufsicht; Fig. 12b: Schnittdarstellung entlang Schnittachse A--A von Fig. 12a);
- Fig. 13: eine maßstabsgerechte Darstellung einer elften Ausführungsform eines erfindungsgemäßen Perfusionsbioreaktors (Fig. 12a: Draufsicht; Fig. 12b: Schnittdarstellung entlang Schnittachse A--A von Fig. 12a); und
- Fig. 14: eine schematische Darstellung einer Ausführungsform eines erfindungsgemäßen Perfusionsbioreaktorsystems.

In den Zeichnungen kennzeichnen dieselben Bezugszeichen jeweils funktionelle gleiche Bestandteile. In den Schnittdarstellungen sind Bestandteile des Perfusionsbioreaktors aus Gummi, beispielsweise Dichtungen, vollständig schwarz gefüllt wiedergegeben, während die übrigen Bestandteile des Perfusionsbioreaktors als geschnittene Teile schraffiert sind. Das in einer Kulturkammer befindliche Gerüstmaterial ist, obwohl geschnitten dargestellt, zur Vereinfachung mit einem Punktmuster wiedergegeben.

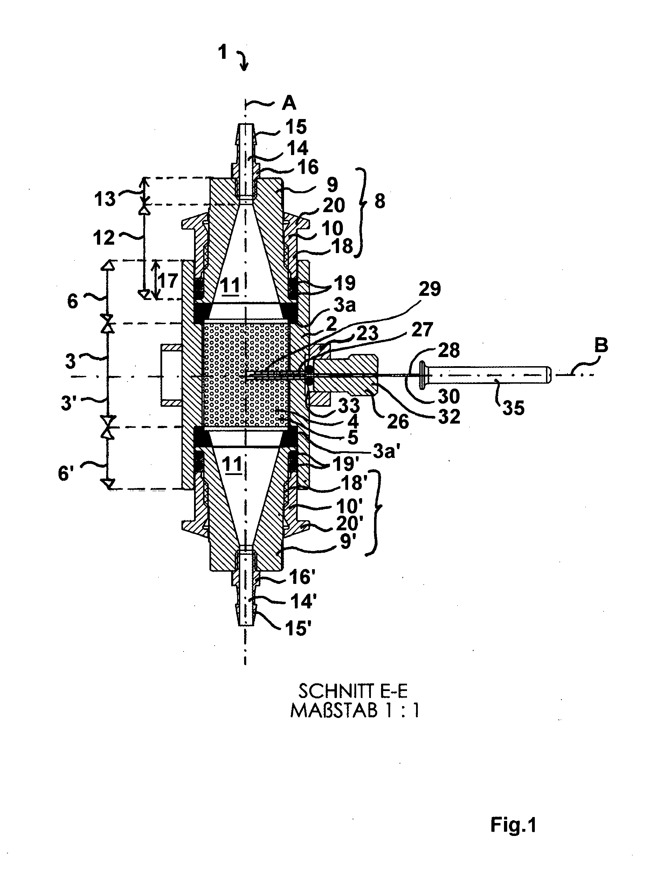

In den Figuren 1 sowie 4 bis 13 sind beide Verschlusselemente jeweils in die Gehäuseöffnungen des Gehäuses eingeführt, wodurch der Innenraum des Gehäuses gegen die Umgebung abgedichtet ist. Die Verschlusselemente können jedoch nach Bedarf unter Öffnung der Gehäuseöffnungen aus den Gehäuseöffnungen gezogen werden. Ebenso ist in den genannten Figuren das Trägerelement für die Sonden auf das Gehäuse aufgesteckt. Es kann jedoch nach Bedarf entfernt werden. Schließlich ist in diesen Figuren die Sondenhalterung bereits in das Trägerelement eingeführt, auch wenn die Sondenhalterung jederzeit aus dem Trägerelement entfernt werden kann. Ebenso kann die Sonde zu einem beliebigen Zeitpunkt angebracht werden.

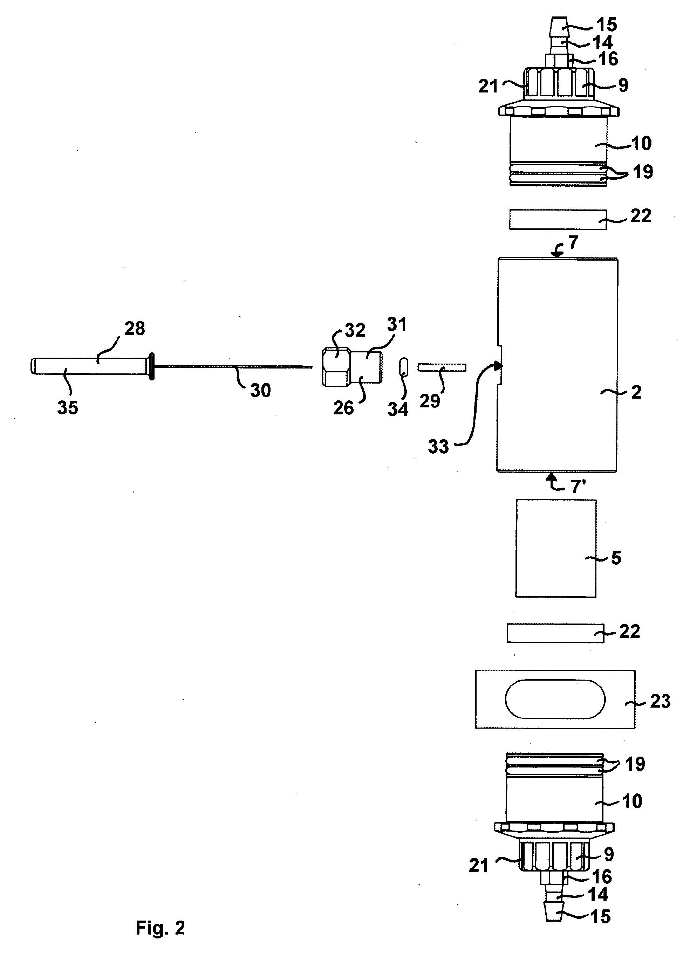

Die in den Figuren 1 bis 3 gezeigte erste Ausführungsform des erfindungsgemäßen Perfusionsbioreaktors 1 umfasst ein zylinderförmiges Gehäuse 2, das einen ersten Abschnitt 3 umfasst, in dem die Kulturkammer 4 ausgebildet ist. In der Kulturkammer 4 befindet sich ein kreiszylinderförmiges Gerüstmaterial 5. Der erste Abschnitt 3 weist einen rohrförmigen Querschnitt auf. Die Innenwand des Gehäuses 2 bildet in ihrem ersten Abschnitt 3 die Mantelfläche der Kulturkammer 4. An die Stirnseiten des ersten Abschnittes 3 grenzen zweite, rohrförmige Abschnitte 6, 6' an, wobei sich die Längsachsen der Abschnitte 3, 6 und 6' auf einer gemeinsamen Geraden, die die Längsachse A des Perfusionsbioreaktors 1 ist, befinden.

Die Außendurchmesser der Abschnitte 3, 6 und 6' sind gleich, die Innendurchmesser der Abschnitte 6 und 6' sind gleich, jedoch größer als der Innendurchmesser des Abschnittes 3. Der Durchmesser der Gehäuseöffnungen 7, 7' entspricht dem Innendurchmesser der zweiten Abschnitte 6, 6'. Aus diesem Grunde ist das Gehäuse 2 einstückig ausgebildet, da das Gerüstmaterial durch einen zweiten Abschnitt 6, 6' in den ersten Abschnitt 3, d. h. die Kulturkammer 4, eingeführt werden und von dort auf demselben Weg wieder entnommen werden kann. Aufgrund des geringeren Innendurchmessers des ersten Abschnittes 3 im Vergleich zu den zweiten Abschnitten 6, 6' entsteht an der Kontaktflächen zwischen dem ersten Abschnitt 3 und den vzweiten Abschnitten 6, 6' jeweils ein Vorsprung 3a, 3a'.

In den Stirnseiten der zweiten Abschnitte 6, 6', die den Stirnseiten des ersten Abschnittes 3 abgewandt sind, und gleichzeitig die Stirnseiten des Gehäuses 2 bilden, befinden sich Gehäuseöffnungen 7, 7', in die Verschlusselemente 8, unter Ausbildung ein dichten Steckverbindung eingesteckt werden können.

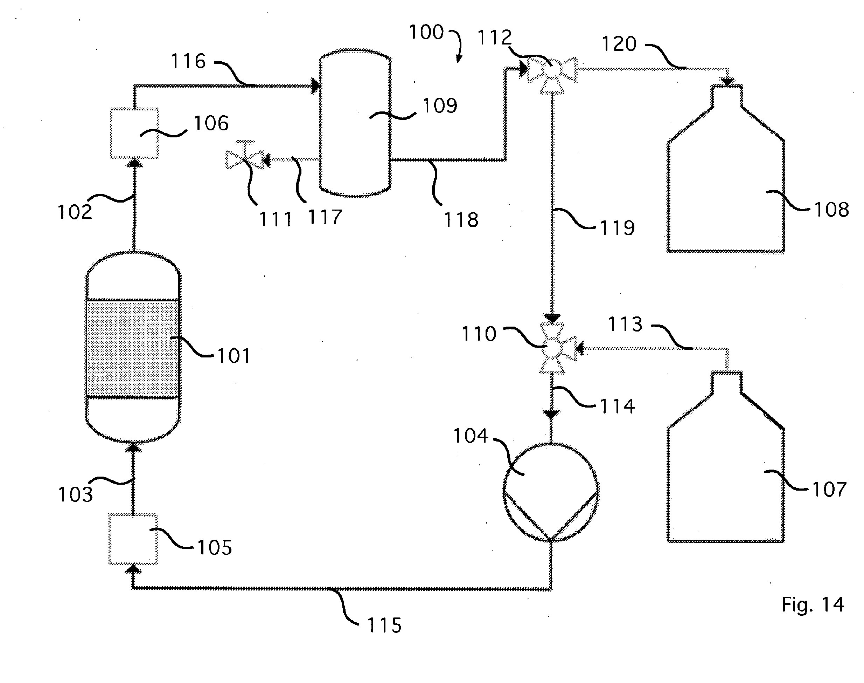

Die beiden Verschlusselemente 8 haben denselben Aufbau. Sie umfassen jeweils einen im Wesentlichen zylinderförmigen Grundkörper 9 und eine Hülse 10, die miteinander verschraubt sind. In dem Grundkörper 9 ist eine kanalartige Öffnung 11 für die Zu- und Abfuhr eines Kulturmediums ausgebildet. Der Grundkörper 9 weist einen sich verjüngenden Abschnitt 12 auf, der sich, wenn das Verschlusselement in die Gehäuseöffnung 7, 7' eingesteckt ist, von der Stirnseite des Verschlusselementes 8, die der Kulturkammer 4 zugewandt ist, in Richtung der Stirnseite des Verschlusselementes 8, die der Kulturkammer 4 abgewandt ist, verjüngt. Die kanalartige Öffnung 11 geht vor dem Erreichen der Stirnseite des Verschlusselementes, die der Kulturkammer abgewandt ist, in einem im wesentlichen kreiszylinderförmigen Abschnitt 13 über, der sich bis zu dieser Stirnseite erstreckt. Dieser Abschnitt 13 weist ein Innengewinde auf, in das ein rohrförmiges Anschlusselement 14 für einen Schlauch 102, 103 (siehe Fig. 14) eingeschraubt ist. Dabei ragt ein Teil des Anschlusselementes 14 aus dem Verschlusselement 8 heraus, wobei auf diesen Teil der Schlauch 102, 103 aufgesteckt werden kann. Dazu weist dieser Teil des Anschlusselementes einen Bereich 15 mit einer sich verjüngenden äußeren Mantelüberfläche auf, der entlang der Längsachse des Anschlusselementes von einem umlaufenden Steg 16 beabstandet ist, dessen äußere Mantelfläche nach Art einer Schraube sechskantförmig ausgebildet ist, so dass er das Einschrauben des Anschlusselementes 14 in den Grundkörper 9 des Verschlusselementes 8 erleichtert.

Die Hülse 10 des Verschlusselementes 8 ist auf dessen Grundkörper 9 lösbar befestigt, wobei sich die äußere Mantelfläche der Hülse in dichtendem Kontakt mit der Innenwand eines zweiten Abschnittes 6, 6' des Gehäuses befindet, wenn das Verschlusselement 8 in eine Gehäuseöffnung 7, 7' des Gehäuses 2 eingeführt ist. Die äußere Mantelfläche der Hülse bildet in diesem Fall die Außenfläche des Einsteckkörpers 17 des Verschlusselementes 8. Diese Außenfläche des Einsteckkörpers ist komplementär zu der Gehäuseöffnung 7, 7'.

Die Hülse 10 ist ringförmig. Zur Befestigung der Hülse 10 auf dem Grundkörper 9 weist die Hülse 10 in ihrer inneren Mantelfläche ein Innengewinde auf, das komplementär zu einem Außengewinde 18 ist, das an der äußeren Mantelfläche des Grundkörpers 9 ausgebildet ist. In der äußeren Mantelfläche der Hülse 10 sind in Umfangsrichtung zur Längsachse der Hülse, die auf der Längsachse A des Gehäuses 2 liegt, zwei umlaufende Nuten zur Aufnahme von Dichtungsringen 19 vorgesehen. Die Nuten sind dabei in der äußeren Mantelfläche in einem Bereich ausgebildet, der beim Einstecken des Verschlusselementes 8 in die Gehäuseöffnung 7, 7' in Kontakt mit der inneren Mantelfläche der Gehäuseöffnung 7, 7' gelangt.

Auf der äußeren Mantelfläche der Hülse 10 ist ein umlaufender Steg 20 ausgebildet, der regelmäßig Einkerbungen in seiner äußeren Mantelfläche aufweist. Ferner weist der Grundkörper 9 Riffelungen auf. Die Länge der Hülse 10 ist, bezogen auf ihre Längsachse, die auf der Längsachse A des Gehäuses 2 liegt, geringer als die Länge des Grundkörpers 9, so dass ein Bereich 21 der Mantelfläche des Grundkörpers 9, der an die Stirnseite des Grundkörpers, die der Kulturkammer 4 angewandt ist, angrenzt, freiliegt. In diesem Bereich können dann Riffelungen und/oder Kerbungen vorgesehen sein.

In dem Gehäuse 2 sind jedem der beiden zweiten Abschnitte 6, 6' ein ringförmiges Vermittlungselement 22 zwischen einer Stirnseite der Kulturkammer 4 und dem dieser Stirnseite zugewandten Verschlusselement 8 angeordnet. Die Vermittlungselemente 22 dienen zur Führung des Kulturmediums in den beiden Abschnitten des Gehäuses zwischen der Stirnseite der Kulturkammer 4 und den kanalartigen Öffnungen 11 der Verschlusselemente 8. Sie vermitteln zwischen dem Durchmesser der kanalartigen Öffnung 11 an der Stirnseite eines Verschlusselementes 8, die der Kulturkammer 4 zugewandt ist, und dem Durchmesser des Gerüstmaterials in der Kulturkammer ist, der größer als der Durchmesser der kanalartigen Öffnung 11 an der Stirnseite eines Verschlusselementes 8 ist. Der Außendurchmesser des ringförmigen Vermittlungselementes 22 ist komplementär zum Innendurchmesser eines zweiten Abschnittes 6, 6'. Der Innendurchmesser des Vermittlungselementes 22 verjüngt sich von der Stirnseite der Kulturkammer in Richtung des zugewandten Verschlusselementes 8, wobei der Innendurchmesser des ringförmigen Vermittlungselementes 21 an der Stirnseite, die an die Stirnseite der Kulturkammer 4 angrenzt, dem dortigen Durchmesser der Kulturkammer und an der Stirnseite, die an das Verschlusselement 8 angrenzt, dem dortigen Durchmesser der kanalartigen Öffnung 11 entspricht.

In der in Figur 1 bis 3 gezeigten Ausführungsform sind die Vermittlungselemente 22 aus einem Silikonmaterial, beispielsweise demselben Material wie die Dichtungsringe 19 gefertigt. Vermittlungselemente 22 sind nicht zwingend erforderlich.

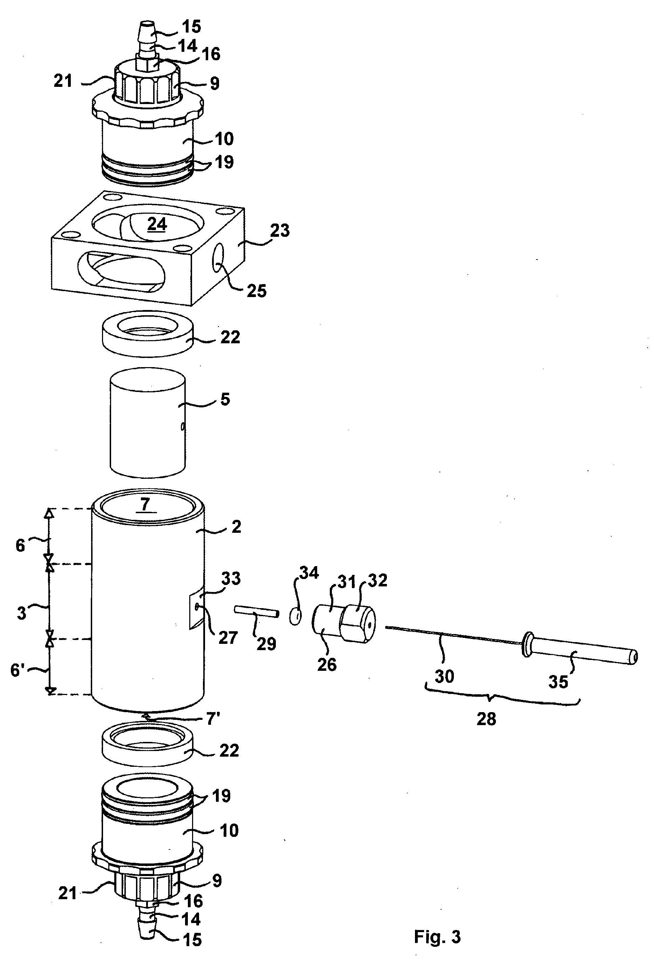

Auf dem Gehäuse 2 sitzt ein Trägerelement 23 für eine Sonde, beispielsweise zur Messung des Sauerstoffpartialdruckes in der Kulturkammer 4, auf. Das Trägerelement 23 weist eine erste Bohrung 24, die komplementär zur Außendurchmesser des Gehäuses 2 ist und deren Längsachse auf der Längsachse A des Gehäuses 2 liegt, und ein zweite Bohrung 25 auf, deren Längsachse orthogonal zur Längsachse der ersten Bohrung verläuft und die Längsachse der ersten Bohrung 24 schneidet. Die Längsachse der ersten Bohrung 24 und die Längsachse der zweiten Bohrung 25 verlaufen durch die geometrische Mitte des Trägerelementes 23 (siehe insbesondere Fig. 3).

In dem Gehäuse 2 ist im Bereich des ersten Abschnittes 3 eine Öffnung 27 (die im Folgenden als Sondenöffnung bezeichnet wird) in der Wandung des Gehäuses 2 ausgebildet, durch die eine Sonde 28 in die Kulturkammer 4 geführt ist. Der Mittelpunkt der Sondenöffnung 27 ist gleichmäßig von beiden Stirnseiten des ersten Abschnittes beabstandet. Der Durchmesser der Sondenöffnung 27 ist komplementär zum Durchmesser eines schlauchartigen Schutzelementes 29, durch das der Messfühler 30 der Sonde 28 geführt ist. Zur Erleichterung der Positionierung des Trägerelementes 23 ist in der Außenseite des Gehäuses 2 im Abschnitt 3 eine Vertiefung 33 vorgesehen, deren Kontur dem Außendurchmesser eines ersten Bereiches 31 der Sondenhalterung 26 entspricht und in der die Sondenöffnung 27 ausgebildet ist. Der Messfühler 30 kann eine optische Faser sein, beispielsweise eine NeedleType-Sonde der Firma PreSens Precision Sensing GmbH, Regensburg, DE.

In die zweite Bohrung 25 ist eine Sondenhalterung 26 eingeführt. Die Sondenhalterung 26 ist ein rohrförmiges Element, durch dessen Hohlraum der Messfühler 30 der Sonde 28 verläuft. Die äußere Mantelfläche der Sondenhalterung weist den ersten Bereich 31 auf, der komplementär zu der zweiten Bohrung 25 in dem Trägerelement 23 ausgebildet ist und sich, wenn die Sondenhalterung 26 in das Trägerelement 23 eingeführt ist, in Kontakt mit der Mantelfläche der zweiten Bohrung 25 befindet, wodurch ein sicherer Halt der Sonde 28 in dem Trägerelement 23 erreicht wird. Die äußere Mantelfläche der Sondenhalterung 26 weist ferner einen zweiten Bereich 32 auf, der an den ersten Bereich 31 angrenzt und nach Art einer Schraube sechskantförmig ausgeformt ist, um das Einstecken der Sondenhalterung 26 in das Trägerelement 23 zu erleichtern.

Ist das Trägerelement 23, wie in Fig. 1 gezeigt, auf das Gehäuse 2 aufgesteckt, so ist die Sondenhalterung 26 derart durch die zweite Bohrung 25 geführt, dass sich die äußere Mantelfläche des ersten Bereiches 31 der Sondenhalterung 26 in Kontakt mit der Mantelfläche der zweiten Bohrung 25 befindet und die Stirnseite der Sondenhalterung 26, die an den ersten Bereich 31 angrenzt, in Kontakt der Außenseite des Gehäuse 2 im Bereich der Vertiefung 33 zugewandt ist. Zwischen dieser Stirnseite der Sondenhalterung und der Vertiefung 33 ist ein Dichtungsring 34 angeordnet, der die Sondenöffnung 27 gegen das Gehäuseäußere abdichtet. Der Dichtungsring 34 besteht zweckmäßigerweise aus demselben Material wie die Dichtungsringe 19. Die Längsachsen der zweiten Bohrung 25, der Sondenhalterung 26, des Schutzelementes 29, des Messfühlers 30, des Dichtungsringes 34 sowie der Sondenöffnung 27 liegen auf einer Gerade, die in Fig. 1 als Querachse B dargestellt ist. Die Querachse B verläuft orthogonal zur Längsachse A und schneidet diese im geometrischen Mittelpunkt der Kulturkammer 4.

Der Messfühler 30 weist an seinem, bezogen auf das Gehäuse 2, distalen Ende ein Griffelement 35 auf.

Die Verwendung des in den Figuren 1 bis 3 gezeigten Perfusionsbioreaktors 1 bis zur Einführung des Gerüstmaterials 5 wird nachstehend an einem Beispiel beschrieben. Dabei liegt der Perfusionsbioreaktor 1 zunächst zerlegt in seine Bestandteile vor, d. h. weder sind die Verschlusselemente 8 in die Gehäuseöffnungen 7, 7' eingesteckt noch ist das Trägerelement 23 auf das Gehäuse 2 aufgesteckt.

Zunächst wird das Gerüstmaterial 5 über die Gehäuseöffnung 7 und den daran angrenzenden zweiten Abschnitt 6 in die Kulturkammer 4 im ersten Abschnitt 3 eingeführt. Anschließend werden ein Vermittlungselement 22 über die Gehäuseöffnung 7 in den angrenzenden Abschnitt 6 eingeführt, so dass das Vermittlungselement 22 auf dem Gerüstmaterial im zweiten Abschnitt 6 aufliegt. Sodann wird in die Gehäuseöffnung 7 ein Verschlusselement 8 eingesteckt, so dass das Verschlusselement an das Vermittlungselement angrenzt. Dann wird über ein weiteres Vermittlungselement 22 über die Gehäuseöffnung 7' in den angrenzenden Abschnitt 6' eingeführt, so dass das Vermittlungselement 22 auf dem Gerüstmaterial 5 im zweiten Abschnitt 6' aufliegt. Sodann wird in die Gehäuseöffnung 7' ein zweites Verschlusselement 8 eingesteckt, so dass das zweite Verschlusselement 8 an das weitere Vermittlungselement 22 angrenzt.

Soll eine Sonde verwendet werden, so sollte vor dem Einbringen des Gerüstmaterials 5 in das Gehäuse 2 eine Bohrung in dem Gerüstmaterial 5 angebracht werden, die sich von der Mantelfläche orthogonal zur Längsachse des Gerüstmaterials 5 bis zu dessen geometrischen Mittelpunkt erstreckt. Bei Einführen des Gerüstmaterials 5 in das Gehäuse muss das Gerüstmaterial dann so ausgerichtet werden, dass die Bohrung im Gerüstmaterial an die Sondenöffnung 27 im Gehäuse 2 angrenzt. Ist das der Fall, kann das Schutzelement 29 über die Sondenöffnung 27 in die Gerüstmaterial-Bohrung eingeführt werden, so dass das Schutzelement 29 sich von der äußeren Mantelfläche des Gehäuses in Richtung des geometrischen Mittelpunktes des Gerüstmaterials erstreckt, wobei sich nicht bis zum geometrischen Mittelpunkt erstrecken sollte, so dass die Messfühlerspitze des später eingeführten Messfühlers 30 im geometrischen Mittelpunkt des Gerüstmaterials 5 freiliegt. Sobald die beiden Gehäuseöffnungen 7,7 verschlossen sind, wird nun das Trägerelement 22 auf das Gehäuse 2 über die erste Bohrung 24 aufgesteckt, so dass sich die zweite Bohrung 25 des Trägerelementes 22 in Höhe der Sondenöffnung 27 befindet. Anschließend wird die Sondenhalterung 26 in die zweite Bohrung 25 eingesetzt und in Richtung des Gehäuses geführt, so dass sie den Dichtungsring 34 gegen die Sondenöffnung 27 presst. Sodann wird der Messfühler 30 durch die Sondenhalterung 27, den Dichtungsring 34 und das Schutzelement 29 bis zum geometrischen Mittelpunkt des Gerüstmaterials 5 geführt, so dass die Messfühlernspitze im geometrischen Mittelpunkt des Gerüstmaterials 5 freiliegt.

Zum Schluss können die beiden Anschlusselemente nun mit den Schläuchen 102, 103 des Perfusionsbioreaktorsystems 100 verbunden werden (siehe Fig. 14), Ist keine Sonde vorgesehen, kann dies unmittelbar nach dem Verschließen der Gehäuseöffnungen 7, 7' erfolgen.

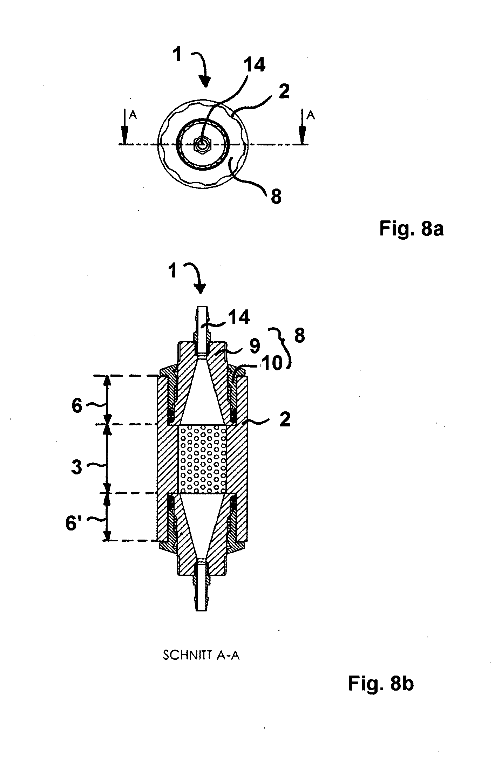

Die in Fig. 1 gezeigte Schnittachse E--E entspricht der Schnittachse A--A in Fig. 8a. Die in Fig. 8a gezeigte Draufsicht der ersten Ausführungsform entspricht der nicht gezeigten Draufsicht der in den Figuren 1 bis 3 gezeigten, ersten Ausführungsform, abgesehen von der Wiedergabe des Trägerelementes und der Sonde.

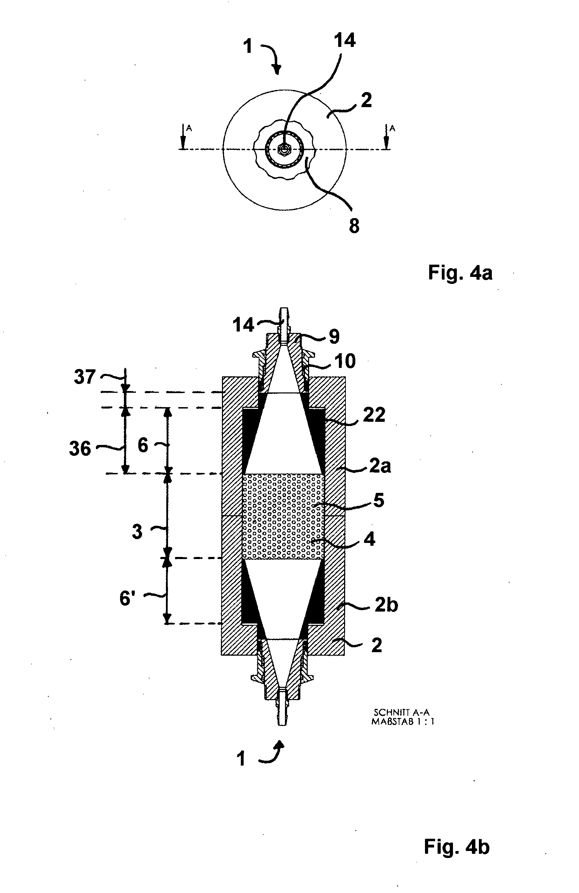

Die in den Figuren 4 bis 13 gezeigten Ausführungsformen entsprechen der in den Figuren 1 bis 3 gezeigten, ersten Ausführungsform mit den nachfolgend aufgeführten Ausnahmen. Auf die Wiedergabe aller Bezugszeichen wurde lediglich zur Verbesserung der Erkennbarkeit der Unterschiede zur ersten Ausführungsform verzichtet. In allen Ausführungsformen weisen die Verschlusselemente 8 denselben Aufbau auf. Auf die Darstellung des Trägerelementes 23 und aller weiteren Bestandteile, die sich auf die Sonde 28 beziehen, einschließlich der Sondenöffnung 27 wurde zur Vereinfachung verzichtet. Selbstverständlich können das Trägerelement und die Bestandteile auch bei den folgenden Ausführungsformen vorgesehen sein.

Das Gehäuse 2 der in Fig. 4 gezeigten Ausführungsform besteht aus zweiten Teilen 2a, 2b, um ein übergroßes Gerüstmaterial 5 in dem erfindungsgemäßen Perfusionsbioreaktor 1 behandeln zu können. Die beiden Teile 2a, 2b sind in einer Ebene, deren Flächennormale parallel zur Längsachse A des Gehäuses 2 und die durch die Kulturkammer 4 verläuft, lösbar miteinander verbunden. Die Innendurchmesser der Abschnitte 3, 6, 6' sind gleich groß, so dass keine Vorsprünge 3a, 3a' ausgebildet sind. Zum Einführen oder zur Entnahme des Gerüstmaterials 5 in die Kulturkammer 4 wird die Verbindung der beiden Teile 2a, 2b gelöst. Anschließend werden die beiden Teile 2a, 2b wieder zu einem Ganzen zusammengefügt. Das Vermittlungselement 22 weist einen ersten Abschnitt 35 auf, dessen Außendurchmesser dem Innendurchmesser des Gehäuses 2 entspricht und der sich von der Stirnseite der Kulturkammer 4 bis zur benachbarten Stirnseite des Gehäuses 2 erstreckt. An diesen ersten Abschnitt 35 schließt sich ein zweiter Abschnitt 36 an, deren Außendurchmesser dem Durchmesser der Gehäuseöffnung 7, 7' entspricht und der sich bis in die Gehäuseöffnung 7, 7' erstreckt. Dort grenzt es an das Verschlusselement 8 an.

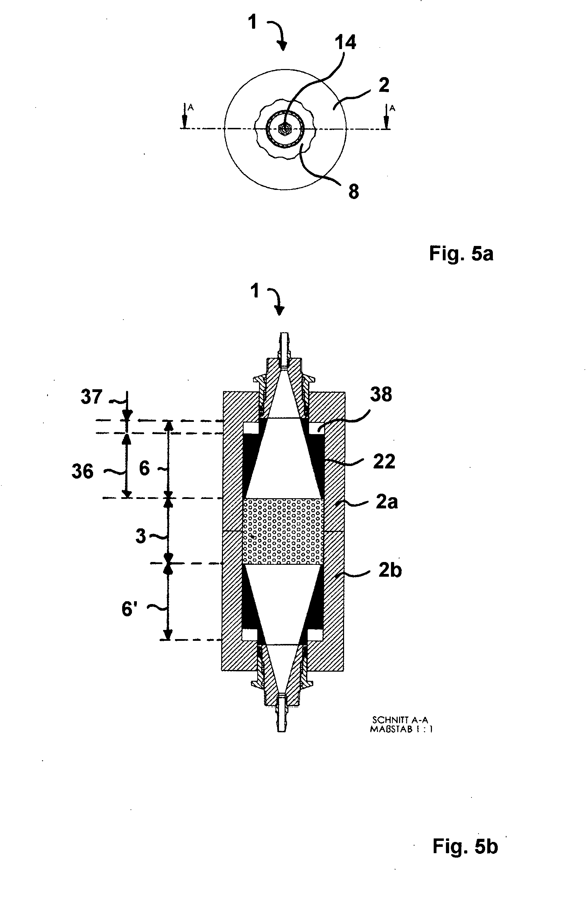

Fig. 5 zeigt eine Ausführungsform des erfindungsgemäßen Perfusionsbioreaktors 1, der für die Behandlung eines Gerüstmaterials 5 angepasst wurde, das im Unterschied zu dem in Fig. 4 gezeigten Gerüstmaterial nur halb solang ist, aber denselben Durchmesser aufweist. Die in Fig. 5 gezeigte Ausführungsform entspricht der in Fig. 4 gezeigten Ausführungsform, außer dass sich der erste Abschnitt 35 des Vermittlungselementes 22, dessen Außendurchmesser dem Innendurchmesser des Gehäuses 2 entspricht, sich von der Stirnseite der Kulturkammer 4 in Richtung der benachbarten Stirnseite des Gehäuses 2 erstreckt, aber nicht an diese angrenzt, sondern von dieser unter Ausbildung eines umlaufenden Hohlraums 38 beabstandet ist. An den ersten Abschnitt 35 schließt sich der zweiter Abschnitt 36 an, deren Außendurchmesser dem Durchmesser der Gehäuseöffnung 7, 7' entspricht und der sich bis in die Gehäuseöffnung 7, 7' erstreckt. Die in Fig. 4 und 5 gezeigten Ausführungsformen des erfindungsgemäßen Perfusionsbioreaktors 1 veranschaulichen die leichte Anpassbarkeit des Perfusionsbioreaktors 1 an Gerüstmaterialien 5 mit unterschiedlichen Längsausdehnungen.

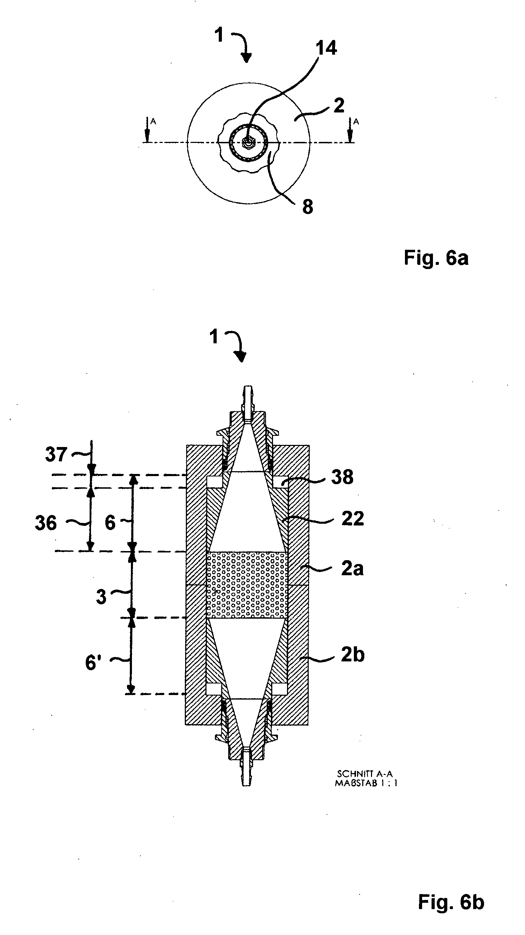

Die in Fig. 6 gezeigte Ausführungsform entspricht der in Fig. 5 gezeigten Ausführungsform, außer dass die Vermittlungselemente 22 nicht aus demselben Material wie die Dichtungsringe 19, 34 gefertigt sind, sondern aus demselben Material wie das Gehäuse 2. Sie sind daher in der Schnittdarstellung (Fig. 6b) nicht schwarz gefüllt, sondern schraffiert dargestellt.

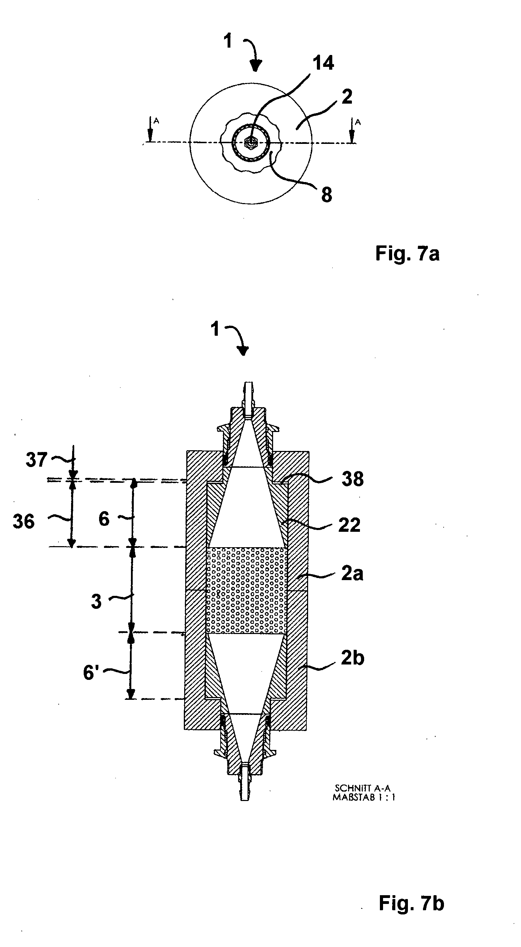

Die in Fig. 7 gezeigte Ausführungsform entspricht der in Fig. 4 gezeigten Ausführungsform, außer dass die Vermittlungselemente 22 nicht aus demselben Material wie die Dichtungsringe 19, 34 gefertigt sind, sondern aus demselben Material wie das Gehäuse 2. Sie sind daher in der Schnittdarstellung (Fig. 7b) nicht schwarz gefüllt, sondern schraffiert dargestellt.

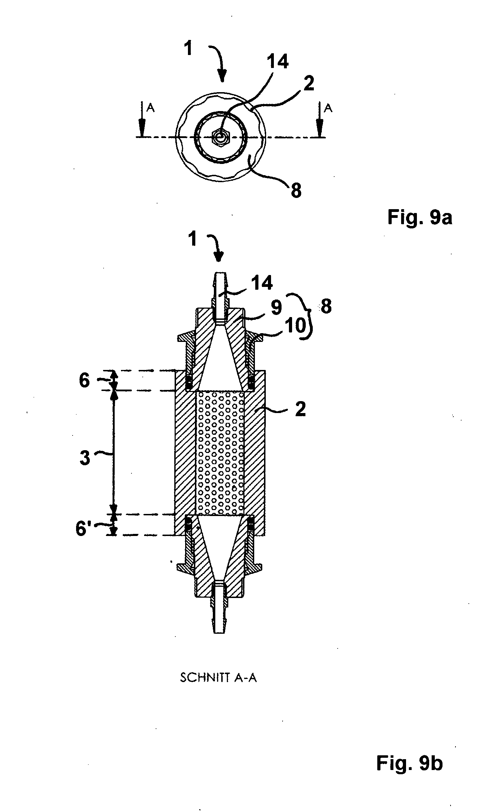

Die in Fig. 8 und 9 gezeigten Ausführungsformen entsprechen der ersten Ausführungsform der Erfindung mit dem Unterschied, dass keine Vermittlungselemente vorgesehen sind. Vielmehr grenzen die Stirnseiten der Kulturkammer 4 untermittelbar an die Verschlusselemente 8 an. Das in Fig. 9 gezeigte Gerüstmaterial 5 ist jedoch deutlich länger als das in Fig. 8 gezeigte Gerüstmaterial 5. Aus diesem Grunde sind die Verschlusselemente 8 in Fig. 9 weniger weit in das Gehäuse 2 eingeführt. Dies veranschaulicht, dass der erfindungsgemäße Perfusionsbioreaktor leicht an Gerüstmaterialien mit unterschiedlichen Längenausdehnung angepasst werden kann, indem die Einstecktiefe der Verschlusselemente 8, bezogen auf die Längsachse A des Gehäuses 2, an die Längenausdehnung des Gerüstmaterials 5 angepasst wird.

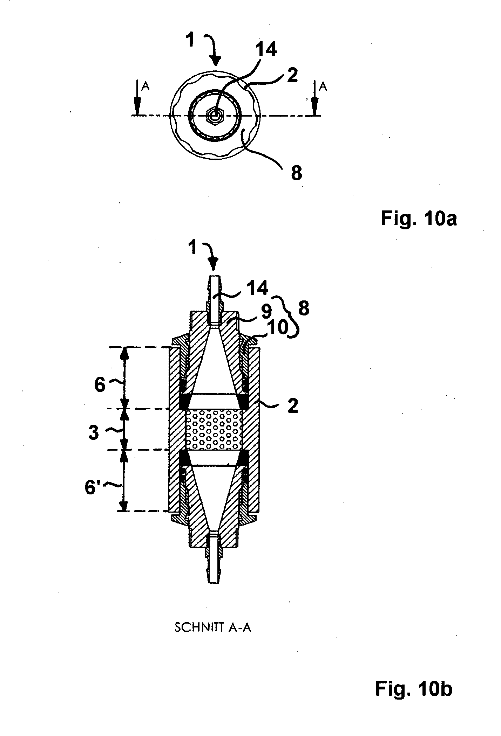

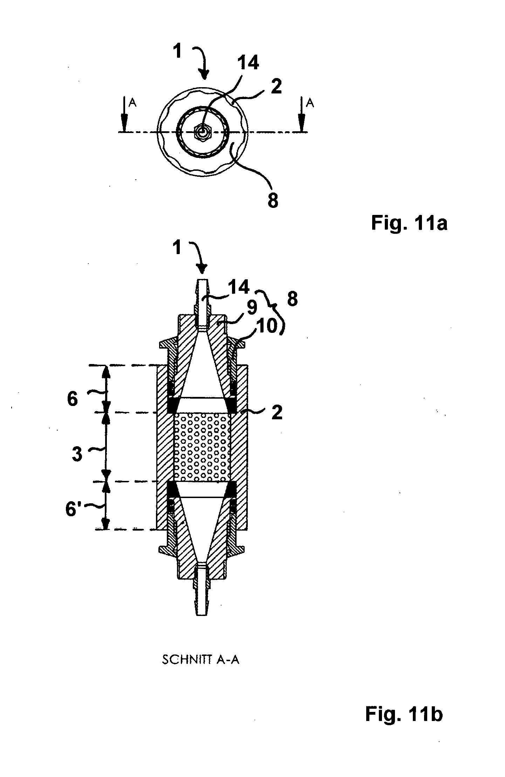

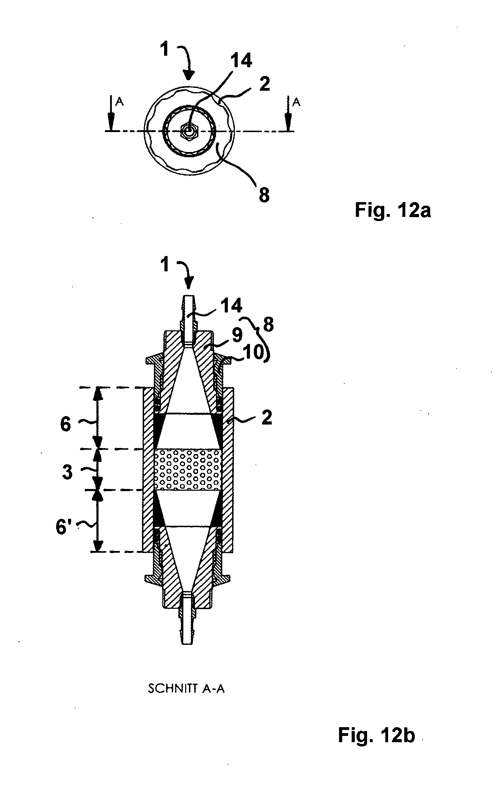

In den Figuren 10 bis 12 wird ebenfalls die leichte Anpassbarkeit des erfindungsgemäßen Perfusionsbioreaktors an unterschiedliche Längenausdehnungen von Gerüstmaterialien veranschaulicht. Die in den Figuren 10 bis 12 gezeigten Ausführungsformen entsprechen der in Fig. 1 gezeigten Ausführungsform. Zu erkennen ist, dass eine Anpassung des Perfusionsbioreaktors an unterschiedliche Längenausdehnungen von Gerüstmaterialien durch eine Veränderung der Einstecktiefe der Verschlusselemente 8, bezogen auf die Längsachse A des Gehäuses 2, erreicht werden kann, wie ein Vergleich von Fig. 10 mit Fig. 11 zeigt, oder durch die Verwendung von Vermittlungselementen 22 mit unterschiedlicher Ausdehnung in axialer Richtung, wie ein Vergleich von Fig. 10 mit Fig. 12 zeigt.

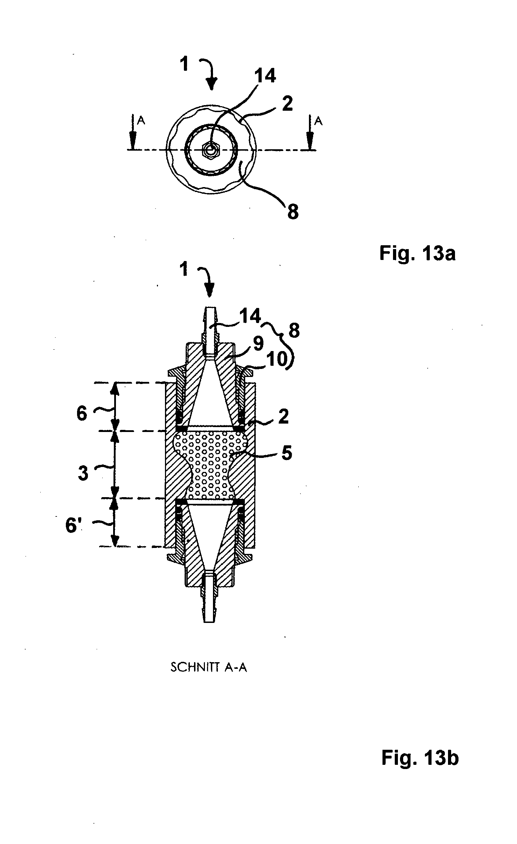

Fig. 13 zeigt eine Ausführungsform eines erfindungsgemäßen Perfusionsbioreaktors, der an die Behandlung eines Gerüstmaterials angepasst ist, dessen Mantelfläche nicht zylinderförmig ist. Zu diesem Zweck ist das Gehäuse 2 im ersten Abschnitt 3 bei der Herstellung des Gehäuses 2 so ausgeformt worden, dass die Innenwandung des Anschnittes 3 komplementär zur Mantelfläche des Gerüstmaterials 5 ist.

In Fig. 14 ist eine Ausführungsform eines Perfusionsbioreaktorsystem 100 vorgesehen, das einen erfindungsgemäßen Perfusionsbioreaktor 101 umfasst. In den Perfusionsbioreaktor 101 ist ein Gerüstmaterial eingebracht, das mit einem Kulturmedium behandelt werden soll. Das System 100 umfasst neben dem Perfusionsbioreaktor 101 eine Pumpe 104, vorzugsweise eine Schlauchpumpe, zur Förderung eines Kulturmediums durch den Perfusionsbioreaktor 101 sowie zwei Durchfluss-Messzellen 105, 106 zur Messung des Sauerstoffpartialdrucks in dem Kulturmedium umfasst, wobei eine erste Durchfluss-Messzelle 105 unmittelbar vor und eine zweite DurchflussMesszelle 106 unmittelbar nach dem Perfusionsbioreaktor 101, bezogen auf die Strömungsrichtung des Systems 100, angeordnet ist. Das erfindungsgemäße Perfusionsbioreaktorsystem 100 umfasst ferner einen Vorratsbehälter 107 für das Kulturmedium, einen Abfallbehälter 108, einen Ausgleichs- und Probennahmebehälter 109 sowie Ventile 110, 111, 112. Die Komponenten sind über Schläuche 102, 103 sowie 113 bis 120 miteinander verbunden.

Das Kulturmedium befindet sich zunächst in einen Vorratsbehälter 107, der über einen Schlauch 113, ein erstes Ventil 110, das ein Dreiwegeventil ist, und Schlauch 114 mit einer Schlauchpumpe 104 verbunden ist. Mittels der Schlauchpumpe 104 wird das Kulturmedium aus dem Verratsbehälter 107 zur ersten Durchfluss-Messzelle 105 über Schlauch 113, 114 und 115 gefördert, wenn das Ventil 110 so geöffnet ist, dass dieser Weg offensteht. Von der Durchfluss-Messzelle 105 gelangt das Kulturmedium über Schlauch 103 zum erfindungsgemäßen Perfusionsbioreaktor 101, tritt in diesen ein, durchströmt das dort befindliche Gerüstmaterial in gezwungener Perfusion und tritt aus dem Perfusionsbioreaktor 101 wieder aus, wodurch es über Schlauch 102 zur zweiten Durchfluss-Messzelle 106 gelangt. Diese zweite Durchfluss-Messzelle ist über einen Schlauch 116 mit dem Ausgleichs- und Probennahmebehälter 109 verbunden, in den das Kulturmedium, über einen Eingang der oberhalb der Ausgänge liegt, eintritt. Das Kulturmedium kann über Schlauch 117, der mit einem Ausgang des Behälters 109 verbunden ist, und Ventil 111, das ein Hahn ist, beispielweise zur Probennahme entnommen werden. Abgesehen davon, fließt das Kulturmedium durch Schlauch 118, der mit einem anderen Ausgang des Behälters 109 verbunden ist, und Ventil 112, das ein Dreiwegeventil ist, zu Ventil 110 zurück. Ist Ventil 110 so gestellt, dass der Weg zur Pumpe 104 offen steht, kann das Kulturmedium im Kreislauf geführt werden. Es ist jedoch ebenso möglich, einen Teil des Kulturmediums über Ventil 112 zu einem Abfallbehälter 108 zu führen und neues Kulturmedium aus dem Vorratsbehälter 107 in den Kulturmedien-Kreislauf einzuspeisen. Die Strömungsrichtung des Kulturmediums innerhalb der Schläuche ist in Fig. 14 durch Pfeile dargestellt.

### Bezugszeichenliste

- 1: Perfusionsbioreaktor
- 2: Gehäuse
- 2a, 2b: Gehäuseteile eines zweiteiligen Gehäuses
- 3: erster Abschnitt des Gehäuses
- 3a, 3a': Vorsprung
- 4: Kulturkammer
- 5: Gerüstmaterial
- 6, 6': zweiter Abschnitt des Gehäuses
- 7, 7': Gehäuseöffnung
- 8: Verschlusselement
- 9: Grundkörper
- 10: Hülse
- 11: Öffnung
- 12: sich verjüngender Abschnitt der Öffnung 11
- 13: kreiszylinderförmiger Abschnitt der Öffnung 11
- 14: Anschlusselement
- 15: sich verjüngende äußere Mantelfläche des Anschlusselementes 14
- 16: Steg
- 17: Einsteckkörper
- 18: Außengewinde
- 19: Dichtungsring
- 20: Steg
- 21: Bereich mit Riffelung
- 22: Vermittlungselement
- 23: Trägerelement
- 24: erste Bohrung
- 25: zweite Bohrung
- 26: Sondenhalterung
- 27: Sondenöffnung
- 28: Sonde
- 29: Schutzelement
- 30: Messfühler
- 31: erster Bereich der Sondenhalterung
- 32: zweiter Bereich der Sondenhalterung
- 33: Vertiefung
- 34: Dichtungsring
- 35: Griffelelement
- 36: erster Abschnitt des Vermittlungselementes 22
- 37: zweiter Abschnitt des Vermittlungselementes 22
- 38: Hohlraum

- 100: Perfusionsbioreaktorsystem
- 101: Perfusionsbioreaktor
- 102: Schlauchverbindung
- 103: Schlauchverbindung
- 104: Pumpe
- 105: erste Durchflussmesszelle
- 106: zweite Durchflussmesszelle
- 107: Vorratsbehälter
- 108: Abfallbehälter
- 109: Ausgleichs- und Probennahmebehälter
- 110: erstes Ventil
- 111: zweites Ventil
- 112: drittes Ventil
- 113 bis 120: Schläuche

## Patentansprüche

1. Perfusionsbioreaktor (1) zum Kultivieren von Zellen auf festen Gerüstmateriallien mit einer Kulturkammer (4) zur Aufnahme des Gerüstmaterials, wobei
- die Kulturkammer, (4) in einem Gehäuse (2) ausgebildet ist und die Mantelfläche der Kulturkammer (4) von der Innenwand des Gehäuse (2) gebildet ist und in den Stirnseiten des Gehäuses (2) Gehäuseöffnungen (7,7') für die Aufnahme von Verschlusselementen (8) gebildet sind, die in die Gehäuseöffnungen (7, 7') dichtend einsteckbar sind,
- die Verschlusselemente (8) kanalartige Öffnungen (11) für die Zu- und Abfuhr eines Kulturmediums aufweisen und die kanalartige Öffnung (11) eines oder beider Verschlusselemente (8) einen Abschnitt aufweist, der sich von der Stirnseite des Verschlusselementes (8), die der Kulturkammer, (4) zugewandt ist, in Richtung der Stirnseite des Verschlusselementes (8), die der Kulturkammer (4) abgewandt ist, verjüngt, und - das Gehäuse (2) einen ersten, zylinderförmigen Abschnitt (3) und zweite, rohrförmige Abschnitte (6, 6') aufweist, die an die Stirnseiten des ersten, zylinderförmigen Abschnittes (3) angrenzen, wobei in dem ersten, zylinderförmigen Anschnitt (3) ein an dessen Stirnseiten offener Hohlraum ausgebildet ist, dessen Wandung die Mantelfläche der Kulturkammer (4) bildet, und wobei die Verschlusselemente (8) über die Gehäuseöffnungen (7, 7') in die zweiten, rohrförmigen Abschnitte (6, 6') einsteckbar sind.

2. Perfusionsbioreaktor nach Anspruch 1, **dadurch gekennzeichnet, dass** die Längsachsen des ersten, zylinderförmigen Abschnittes (3) und der zweiten, rohrförmigen Abschnitte (6, 6') auf einer Geraden liegen, die Innendurchmesser der zweiten Abschnitte (6 , 6') im Verhältnis zu dem des ersten Abschnittes (3) gleich oder ungleich sind.

3. Perfusionsbioreaktor nach einem der vorstehenden Ansprüche, **dadurch gekennzeichnet, dass** zumindest eines der Verschlusselemente (8) einen Einsteckkörper (17) mit einer Außenfläche aufweist, die komplementär zu einer Gehäuseöffnung (8) ausgebildet ist und zumindest ein Dichtungselement (19) zur Abdichtung der Grenzfläche zwischen der Außenfläche des Einsteckkörpers (17) und der inneren Mantelfläche der Gehäuseöffnung (7, 7") trägt.

4. Perfusionsbioreaktor nach einem der vorstehenden Ansprüche, **dadurch gekennzeichnet, dass** zumindest eines der Verschlusselemente (8) zweiteilig ausgebildet ist, wobei das erste Teil ein im Wesentlichen zylinderförmiger Grundkörper (9) ist, in dem die kanalartige Öffnung (11) für die Zu- und Abfuhr eines Kulturmediums ausgebildet ist, und das zweite Teil ein Hülse (10) ist, die auf dem Grundkörper (9) lösbar befestigt ist und deren äußere Mantelfläche sich in dichtendem Kontakt mit dem Gehäuse (2) befindet, wenn das Verschlusselement (8) in das Gehäuse (2) eingesteckt ist.

5. Perfusionsbioreaktor nach einem der vorstehenden Ansprüche, **dadurch gekennzeichnet, dass** er ferner ein Trägerelement (23) für eine Sonde (28) umfasst, wobei das Trägerelement (28) auf das Gehäuse (2) derart aufsteckbar ist, dass die Sonde (28) durch eine Sondenöffnung (27) in dem Gehäuse (2) in die Kulturkammer (4) eingeführt werden kann.

6. Perfusionsbioreaktor nach Anspruch 5, **dadurch gekennzeichnet, dass** das Trägerelement (23) eine erste Bohrung (24), die komplementär zur Außenwand des Gehäuses (2) zumindest im Bereich der Kulturkammer (4) ist, und ein zweite Bohrung (25) aufweist, deren Längsachse orthogonal zur Längsachse der ersten Bohrung verläuft, wobei eine Sondenhalterung (26) durch die zweite Bohrung (25) geführt ist.

7. Perfusionsbioreaktor nach einem der vorstehenden Ansprüche, **dadurch gekennzeichnet, dass** das Gehäuse (2) ein- oder zweiteilig ist, wobei ein zweiteiliges Gehäuse (2) ein erstes (2a) und ein zweites Gehäuseelement (2b) umfasst, die im Bereich der Kulturkammer, (4) miteinander trennbar verbunden sind.

8. Perfusionsbioreaktor nach einem der vorstehenden Ansprüche, **dadurch gekennzeichnet, dass** in dem Gehäuse (2) ein oder mehrere ringförmige Vermittlungselemente (22) jeweils zwischen einer Stirnseite der Kulturkammer, (4) und dem dieser Stirnseite zugewandten Verschlusselement (8) angeordnet sind, die zur Führung des Kulturmediums zwischen der Stirnseite der Kulturkammer (4) und den kanalartige Öffnungen (11) der Verschlusselemente (8) dienen.

9. Perfusionsbioreakter nach Anspruch 8, **dadurch gekennzeichnet, dass** sich der Innendurchmesser eines Vermittlungselementes (22) von der Stirnseite der Kulturkammer (4) in Richtung des zugewandten Verschlusselementes (8) verjüngt, wobei der Innendurchmesser des ringförmigen Vermittlungselementes (22) an der Stirnseite, die an die Stirnseite der Kulturkammer (4) angrenzt, dem dortigen Durchmesser der Kulturkammer (4) und an der Stirnseite, die an das Verschlusselement (8) angrenzt, dem dortigen Durchmesser der kanalartigen Öffnun (11) entspricht.

10. Perfusionsbioreaktor nach einem der vorstehenden Ansprüche, **dadurch gekennzeichnet, dass** zumindest das Gehäuse (2), die Verschlusselemente (8) und, falls vorgesehen, die Dichtungen (19) und Vermittlungselemente (22) aus einem autoklavierbaren Kunststoffmaterial gefertigt sind.

11. Perfusionsbioreaktorsystem (100), umfassend einen Perfusionsbioreaktor (101) nach einem der Ansprüche 1 bis 10, eine Pumpe (104) zur Förderung eines Kulturmediums durch den Perfusionsbiareaktor (101) sowie zumindest einer Durchfluss-Messzelle (105) zur Messung des Sauerstoffpartialdrucks in dem Kulturmedium, wobei die Durchfluss-Messzelle (105) unmittelbar vor oder nach dem Perfusionsbioreaktor (101), bezogen auf die Strömungsrichtung des Reaktors (105), angeordnet ist.

## Claims

1. A perfusion bioreactor (1) for culturing cells on solid skeleton materials that has a culture chamber (4) for receiving the skeleton material, wherein
- the culture chamber (4) is formed in a housing (2) and the circumferential surface of the culture chamber (4) is formed from the inside wall of the housing (2) and housing openings (7, 7') are formed in the front sides of the housing (2) for receiving closing elements (8) that can be inserted into the housing openings (7, 7') in a sealing manner;
- the closing elements (8) have channel-like openings (11) for supplying and discharging a culture medium and the channel-like opening (11) of one or both closing elements (8) has a portion that tapers from the front side of the closing element (8) that faces the culture chamber (4) towards the front side of the closing element (8) that is turned away from the culture chamber (4); and
- the housing (2) has a first cylindrical portion (3) and second tubular portions (6, 6') that abut on the front sides of the first cylindrical portion (3), wherein in the first cylindrical portion (3) there is formed a cavity that is open at its front sides, the wall of which forms the circumferential surface of the culture chamber (4), and wherein the closing elements (8) can be inserted into the second tubular portions (6,6') via the housing openings (7, 7').

2. The perfusion bioreactor according to claim 1, **characterized in that** the longitudinal axes of the first cylindrical portion (3) and the second tubular portions (6, 6') are on a straight line, the inner diameters of the second portions (6, 6') are equal or unequal in relation to that of the first portion (3).

3. The perfusion bioreactor according to anyone of the preceding claims, **characterized in that** at least one of the closing elements (8) has an inserting body (17) with an outer surface that is formed complementary to a housing opening (8) and bears at least one sealing element (19) for sealing the interface between the outer surface of the inserting body (17) and the inner circumferential surface of the housing opening (7, 7').

4. The perfusion bioreactor according to anyone of the preceding claims, **characterized in that** at least one of the closing elements (8) is formed in two parts, wherein the first part is a substantially cylindrical main body (9) in which the channel-like opening (11) for supplying and discharging a culture medium is formed, and the second part is a sleeve (10) that is detachably attached to the main body (9) and the outer circumferential surface of which is in a sealing contact with the housing (2) in a state where the closing element (8) is inserted into the housing (2).

5. The perfusion bioreactor according to anyone of the preceding claims, **characterized in that** it further comprises a support element (23) for a probe (28), wherein the support element (28) can be fitted on the housing (2) such that the probe (28) can be inserted into the culture chamber (4) through a probe opening (27) in the housing (2).

6. The perfusion bioreactor according to claim 5, **characterized in that** the support element (23) has a first bore (24) complementary to the outside wall of the housing (2) at least in the area of the culture chamber (4) and a second bore (25) the longitudinal axis of which extends orthogonally to the longitudinal axis of the first bore, wherein a probe holder (26) is passed through the second bore (25).

7. The perfusion bioreactor according to anyone of the preceding claims, **characterized in that** the housing (2) is one-part or two-part, wherein a two-part housing (2) comprises a first (2a) and a second housing element (2b) that are detachable connected to each other in the area of the culture chamber (4).

8. The perfusion bioreactor according to anyone of the preceding claims, **characterized in that** in the housing (2) one or more annular mediating element(s) (22) each are arranged between a front side of the culture chamber (4) and the closing element (8) facing said front side that serve for leading the culture medium between the front side of the culture chamber (4) and the channel-like openings (11) of the closing elements (8).

9. The perfusion bioreactor according to claim 8, **characterized in that** the inner diameter of one mediating element (22) tapers from the front side of the culture chamber (4) towards the facing closing element (8), wherein the inner diameter of the annular mediating element (22) at the front side, abutting on the front side of the culture chamber (4), corresponds to the diameter of the culture chamber (4) there and at the front side, abutting on the closing element (8), corresponds to the diameter of the channel-like opening (11) there.

10. The perfusion bioreactor according to anyone of the preceding claims, **characterized in that** at least the housing (2), the closing elements (8) and if provided the seals (19) and mediating elements (22) are made of an autoclavable plastic material.

11. A perfusion bioreactor system (100), comprising a perfusion bioreactor (101) according to anyone of claims 1 to 10, a pump (104) for conveying a culture medium through the perfusion bioreactor (101) as well as at least one continuous-flow cell (105) for measuring the oxygen partial pressure in the culture medium, wherein the continuous-flow cell (105) is arranged immediately upstream or downstream of the perfusion bioreactor (101) with respect to the flow direction of the reactor (105).

## Revendications

1. Bioréacteur à perfusion (1) pour la culture de cellules sur des matériaux de structure fixes comportant une chambre de culture (4) pour loger le matériau de culture,
- la chambre de culture (4) étant réalisée dans un carter (2) et la surface extérieure de la chambre de culture (4) étant formée par la paroi intérieure du carter (2) et des ouvertures de carter (7, 7') étant ménagées dans les faces frontales du carter (2) pour loger des éléments de fermeture (8) pouvant être introduits hermétiquement dans les ouvertures de carter (7, 7'),
- les éléments de fermeture (8) présentant des ouvertures de type conduit (11) pour l'amenée et l'évacuation d'un milieu de culture, et une ouverture de type conduit (11) d'un ou de deux éléments de fermeture (8) présentant une section qui s'amincit de la face frontale de l'élément de fermeture (8) orientée vers la chambre de culture (4) en direction de la face frontale de l'élément de fermeture (8) opposée à la chambre de culture (4), et
- le carter (2) présentant une première section de forme cylindrique (3) et des deuxièmes sections en forme de tube (6, 6') jouxtant les faces frontales de la première section de forme cylindrique (3), dans la première section de forme cylindrique (3) étant réalisé un espace creux ouvert à ses faces frontales dont la paroi forme la surface extérieure de la chambre de culture (4) et les éléments de fermeture (8) pouvant être introduits dans les deuxièmes sections en forme de tube (6, 6') en passant par les ouvertures de carter (7, 7').

2. Bioréacteur à perfusion selon la revendication 1 **caractérisé en ce que** les axes longitudinaux de la première section de forme cylindrique (3) et des deuxièmes sections en forme de tube (6, 6') se trouvent sur une droite et que les diamètres intérieurs des deuxièmes sections (6, 6') sont identiques ou non identiques par rapport à celui de la première section (3).

3. Bioréacteur à perfusion selon l'une des revendications précédentes **caractérisé en ce qu'**au moins l'un des éléments de fermeture (8) présente un corps enfichable (17) avec une surface extérieure qui est réalisée en plus d'une ouverture de carter (8) et porte au moins un élément d'étanchéité (19) à l'étanchéité de la surface limite entre la surface extérieure du corps enfichable (17) et la surface intérieure de l'ouverture de carter (7, 7').

4. Bioréacteur à perfusion selon l'une des revendications précédentes **caractérisé en ce qu'**au moins l'un des éléments de fermeture (8) est réalisé en deux parties, la première partie étant un corps de base (9) pour l'essentiel en forme de cylindre dans lequel l'ouverture de type conduit (11) est formé pour l'amenée et l'évacuation d'un milieu de culture et la deuxième partie étant une douille (10) fixée sur le corps de base (9) de façon à pouvoir être détachée et dont la surface extérieure se trouve en contact hermétique avec le carter (2) lorsque l'élément de fermeture (8) est introduit dans le carter (2).

5. Bioréacteur à perfusion selon l'une des revendications précédentes **caractérisé en ce qu'**il comprend en plus un élément porteur (23) pour une sonde (28), l'élément porteur (28) pouvant être enfiché sur le carter (2) de façon que la sonde (28) puisse être introduite dans la chambre de culture (4) par une ouverture de sonde (27) placée dans le carter (2).

6. Bioréacteur à perfusion selon la revendication 5 **caractérisé en ce que** l'élément porteur (23) présente un premier trou (24), qui est complémentaire à la paroi extérieure du carter (2) au moins dans la zone de la chambre de culture (4), et un deuxième trou (25) dont l'axe longitudinal passe de façon orthogonale par rapport à l'axe longitudinal du premier trou, un support de sonde (26) étant guidé par le deuxième trou (25).

7. Bioréacteur à perfusion selon l'une des revendications précédentes **caractérisé en ce que** le carter (2) est en une ou deux parties, un carter (2) en deux parties comprenant un premier (2a) et un deuxième élément de carter (2b) qui sont associées entre eux de façon à pouvoir être séparées dans la zone de la chambre de culture (4).

8. Bioréacteur à perfusion selon l'une des revendications précédentes **caractérisé en ce que** dans le carter (2) sont placés un ou plusieurs éléments de commutation (22) annulaires respectivement entre une face frontale de la chambre de culture (4) et l'élément de fermeture (8) orienté vers cette face frontale qui servent à guider le milieu de culture entre la face frontale de la chambre de culture (4) et les ouvertures de type conduit (11) des éléments de fermeture (8).

9. Bioréacteur à perfusion selon la revendication 8 **caractérisé en ce que** le diamètre intérieur d'un élément de commutation (22) s'amincit de la face frontale de la chambre de culture (4) en direction de l'élément de fermeture (8) orienté dans ce sens, le diamètre intérieur de l'élément de commutation (22) annulaire à la face frontale qui jouxte la face frontale de la chambre de culture (4) correspondant au diamètre correspondant de la chambre de culture (4) et à la face frontale qui jouxte l'élément de fermeture (8), au diamètre correspondant de l'ouverture de type conduit (11).

10. Bioréacteur à perfusion selon l'une des revendications précédentes **caractérisé en ce qu'**au moins le carter (2), les éléments de fermeture (8) et, si prévu, les joints d'étanchéité (19) et les éléments de commutation (22) sont fabriqués à partir d'un matériau plastique pouvant être autoclavé.

11. Système de bioréacteur à perfusion (100), comprenant un bioréacteur à perfusion (101) selon l'une des revendications 1 à 10, une pompe (104) pour véhiculer un milieu de culture à travers le bioréacteur à perfusion (101) ainsi qu'au moins une cellule de mesure de débit (105) pour mesurer la pression partielle d'oxygène dans le milieu de culture, la cellule de mesure de débit (105) étant placée directement avant ou après le bioréacteur à perfusion (101), en référence au sens d'écoulement du réacteur (105).
